# EUROPEAN PATENT APPLICATION

(11) **EP 2 246 432 A2**
(43) Date of publication of application: **03.11.2010**
(21) Application number: 09705069.4
(22) Date of filing: 29.01.2009
(51) Int. Cl.: C12N 15/11

(54) **METHODS AND COMPOSITIONS CAPABLE OF CAUSING POST-TRANSCRIPTIONAL SILENCING OF GENE EXPRESSION IN A SYNERGIC MANNER**

(30) Priority: 29.01.2008 ES 200800222
(71) Applicant: Proyecto de Biomedicina Cima, S.L., 31180 Cizur Mayor - Navarra (ES)
(72) Inventor: ABAD LLORET, Xabier, E-31008 Pamplona - Navarra (ES); FORTES ALONSO, María Purificación, E-31008 Pamplona - Navarra (ES)
(74) Representative: ABG Patentes, S.L.
(86) International application number: PCT/ES2009/000045
(87) International publication number: WO 2009/095517

(57) **Abstract**

The invention relates to compositions for the post-transcriptional inhibition of gene expression by means of the combined use of a modified U1 snRNA targeted at a pre-selected region in a target pre-mRNA and of a gene expression-silencing agent of the siRNA, shRNA and/or miRNA type, as well as the use of said combinations for the treatment of diseases caused by the unwanted over-expression of a protein.

## Description

### TECHNICAL FIELD OF THE INVENTION

The invention relates to methods for the silencing of gene expression in a post-transcriptional manner and, more specifically, to compositions which allow achieving higher silencing levels than those obtained using the components of said compositions separately.

### BACKGROUND OF THE INVENTION

The efficient and specific inhibition of gene expression is the basis of some gene therapy protocols and functional gene studies. The expression of certain genes is sometimes toxic for the cell, generating diseases such as the expression of viral genes in infected cells, which are dominant negative in hereditary diseases, or genes the expression of which should be restricted, such as oncogenes. The inhibition of the expression of these toxic genes would allow curing the diseases they trigger. In addition, genomic and proteomic studies allow isolating a large number of genes, some of which have an unknown function. These techniques allow classifying these genes either according to their expression levels or according to the conditions regulating such expression, but do not say much about their function. The systematic and specific inhibition of the expression of these genes would allow developing simple protocols to determine their function.

The simplest way of specifically inhibiting the expression of a gene is to reduce the stability of the RNA that is transcribed from it or to block its translation into protein. The most used classic systems based on antisense technology (antisense ribozymes and oligonucleotides) have not had much success, perhaps because in order to function they need to interact with "*in vivo*" target RNAs and these are customarily coated with proteins and forming secondary and tertiary structures protecting them from these interactions.

Another alternative to specifically inhibit the expression of a gene is by means of decreasing the stability of the RNA using RNA interference. This technique allows promoting the specific degradation of the gene of interest by means of using so-called small interfering RNAs (siRNAs), consisting of an RNA duplex made up of two complementary RNA strands with a length of 21-22 nucleotides with projecting 3' ends. (Elbashir, S.M. et al., Genes Dev. 2001, 15:188-200). siRNAs hybridize with complementary sequence mRNAs in a multi-protein complex called RISC, where the degradation of the mRNA takes place. However, the gene silencing methods based on the use of siRNAs are complicated by the high cost of the preparation of siRNAs by means of chemical synthesis, especially when large amounts are needed for their use in animal models or, in the future, in humans. To avoid the problem associated to the high cost of siRNA synthesis, strategies have been developed which allow siRNAs to be produced in the actual cell from shRNAs. However, this technique still has to be developed to facilitate the selection of sequences assuring the highest specificity and the lowest toxicity. Furthermore, the use of siRNA is made more difficult by the excessive experimentation required to identify the optimum site of the target mRNA such that the siRNA which will hybridize with it has the highest specificity and the lowest toxicity, and by the side effects such as the activation of the response to interferon and the "off-targets", where the bindings of the RNA strand that are not completely complementary (non-specific) to transcripts different from the target cause a series of unwanted effects.

An alternative may be the use of the functional properties of small nuclear ribonucleoproteins (snRNPs), some of which interact with specific sequences of the pre-mRNAs and participate in different stages of the maturation process of the mRNA in the nucleus. In the case of U1 snRNP, it is formed by the proteins U1A, 70K, U1C and seven Sm proteins bound to an RNA molecule (U1 snRNA). The 5' end of its RNA hybridizes by base pairing with the 5' end of the sequences that are going to be eliminated, the introns, at the splicing site of the pre-mRNA. This interaction is stabilised by proteins interacting directly or indirectly with U1 snRNA and with the mRNA that is going to be processed. Once U1snRNP interacts with the pre-mRNA, a series of bindings of other proteins and snRNPs are triggered, which will determine the elimination of the intron. For this to be possible, it is necessary that the U1 snRNP separates from the pre-mRNA.

Furthermore, when U1 snRNP binds to RNA, it inhibits the action of the processing and polyadenylation machinery on nearby sequences. Protein 70K of U1 snRNP is capable of binding to the carboxy-terminal end of the poly(A) polymerase (PAP) subunit of the polyadenylation complex, inhibiting its function (Gunderson et al., 1997, Genes & Dev. 11:761-773.).

The inhibition exerted by U1 snRNP on the processing and polyadenylation machinery has been used to control gene expression. It has thus been described that it is possible to modify the region of U1 snRNAs involved in the recognition of the intronic splicing region such that it binds specifically to the 3' terminal exon of a target mRNA, resulting in the degradation of said target mRNA. This method has been applied to decrease the expression of reporter genes (US20030082149, Beckley, S.A. et al., 2001, Mol. Cell. Biol., 21:2815-2825, Furth, P.A. et al., 1994, Mol. Cell. Biol., 14:5278-5289, Fortes, P. et al., 2003, Proc. Natl. Acad. Sci. USA, 100:8264-8269 and EP1384784) and of endogenous genes (Furth, P.A. et al., *supra*, Fortes, P. et al., *supra*, and Liu, P et al., 2004, Nucleic Acid Res., 32:1512-1517). Sajic et al., (Nucleic Acids Res., 2007, 35:247-255) have described the capacity of a modified U1 specific for the terminal exon of the viral HIV-1 RNA to inhibit the expression of the transcript derived from the HIV-1 genome and, therefore, to inhibit the replication of said virus.

However, the possibility of using said U1 snRNAs to inhibit the expression of a target gene *in vivo* is limited by the difficulty of finding good target sequences allowing maximum inhibitions with the highest specificity and the lowest toxicity. Therefore, and as occurs in the case of RNAi, there is a need for improvements in the gene silencing method based on U1 snRNA which allow reaching effective silencing levels at low intracellular concentrations of U1, such that their toxicity is reduced and the application of said silencing methods *in vivo* is also possible.

### SUMMARY OF THE INVENTION

The authors of the present invention have shown that, surprisingly, the combined use of modified U1 snRNAs specific for a certain target gene and gene expression-silencing agents directed to the same target gene results in an inhibition of the expression of the target gene greater than that observed when each of the agents is used separately.

Thus, in a first aspect, the invention relates to a composition comprising one or several containers or a kit-of-parts which comprises:
(i) a first component comprising at least an U1 snRNA or a polynucleotide encoding an U1 snRNA, wherein said U1 snRNA is modified in its binding sequence to the GU consensus sequence of the 5' end of the intron such that it binds specifically to a pre-selected region of the 3' terminal exon of a target pre-mRNA and which is capable of inhibiting the maturation of said target pre-mRNA; and
(ii) a second component comprising at least one gene expression-silencing agent targeted specifically at a pre-selected region of the mRNA resulting from the processing of the pre-mRNA which is the target of the U1 snRNA as defined in (i) and which is capable of causing the *in vivo* silencing of said target mRNA.

In a second aspect, the invention relates to a A polynucleotide comprising
(i) a sequence encoding at least one U1 snRNA modified in its binding sequence to the GU consensus sequence of the 5' end of the intron such that it binds specifically to a pre-selected region of the 3' terminal exon of a target pre-mRNA and which is capable of inhibiting the processing of the target pre-mRNA, and
(ii) a sequence encoding a silencing agent targeted specifically at a pre-selected region of the mRNA resulting from the processing of the pre-mRNA which is the target of the U1 snRNA encoded by the polynucleotide defined in (i) and which is capable of causing the *in vivo* silencing of said target mRNA.

In additional aspects, the invention relates to an expression vector comprising a polynucleotide of the invention, a cell comprising a vector of the invention and a transgenic animal comprising a polynucleotides of the invention.

In another aspect, the invention relates to a composition or kit of the invention with a polynucleotide of the invention, with a vector of the invention or with a cell of the invention for its use as a medicament.

In another aspect, the invention relates to a composition or kit of the invention with a polynucleotide of the invention, with a vector of the invention or with a cell of the invention for the treatment or the prevention of infectious, tumor, neoplastic or neurodegenerative diseases.

In another aspect, the invention relates to a non-therapeutic method for the *in vitro* or *in vivo* post-transcriptional inhibition of the expression of a target gene in a biological system comprising putting said system in contact with a composition or kit of the invention with a polynucleotide of the invention, with a vector of the invention or with a cell of the invention for the treatment or the prevention of infectious, tumor, neoplastic or neurodegenerative diseases.

### BRIEF DESCRIPTION OF THE DRAWINGS

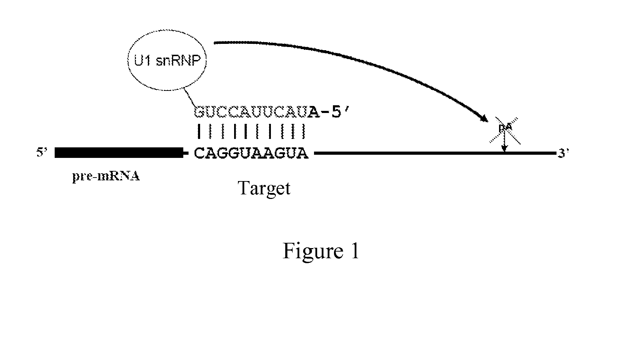

**Figure 1****. Representative scheme of the RNAu inhibition mechanism.** The 5' end of the snRNA of a U1 snRNP molecule has been modified such that its sequence is complementary to a pre-selected region of the 3' terminal exon of a pre-mRNA target. When the U1 snRNP molecule binds to said pre-selected sequence by base pairing, it causes the inhibition of the polyadenylation (pA) of the target RNA and, therefore, the inhibition of the expression of the gene. The U1 snRNA molecules modified according to this scheme are also called U1i.

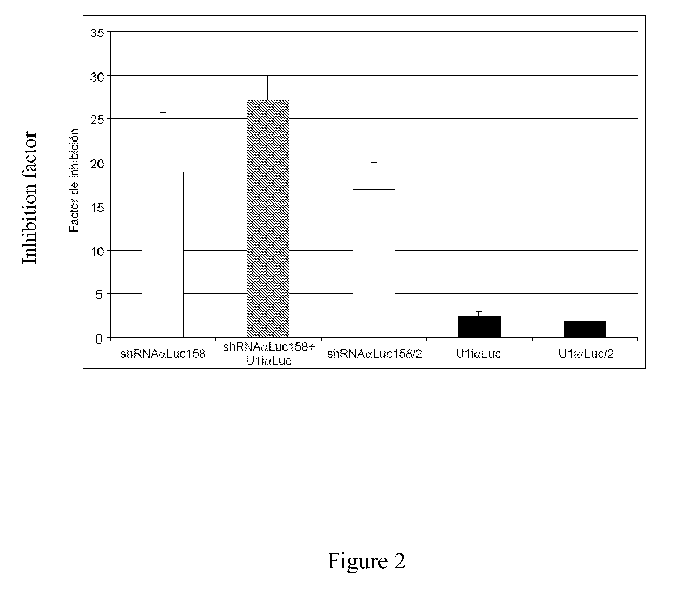

**Figure 2****. The inhibition of the expression of a reporter gene using a strong shRNA synergizes with the inhibition of its expression using U1i.** The luciferase activity in cells transfected with a control plasmid has been calculated with one or half a dose of a plasmid expressing the shRNA against the luciferase target sequence 158 (shRNAαLuc158 and shRNAαLuc158/2, respectively), with one or a half dose of a plasmid expressing a U1i against luciferase (U1iαLuc and U1iαLuc/2, respectively), and with half a dose of shRNAαLuc158 and half a dose of U1iαLuc (shRNAαLuc158/2 + U1iαLuc/2). The inhibition factor, which is the result of dividing the luciferase activity of the control (non-inhibited) cells by the luciferase activity obtained in the rest of the cases, is represented. The error bars represent the standard deviation of 9 independent measurements.

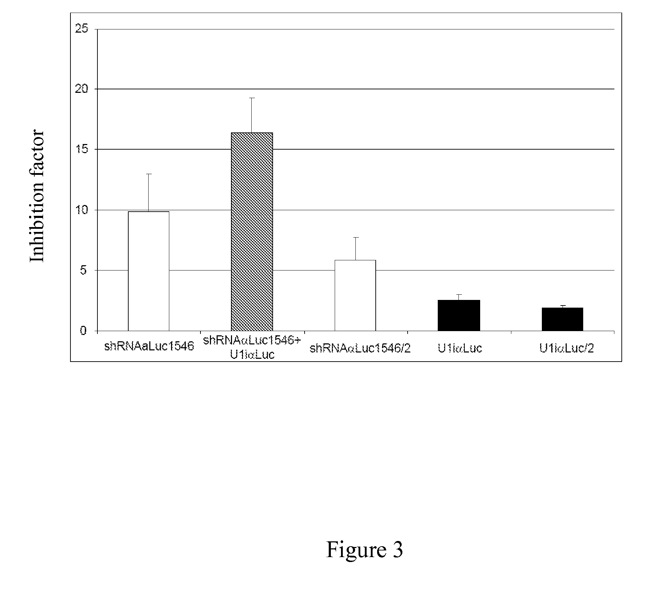

**Figure 3****. The inhibition of the expression of a reporter gene using strong shRNAs synergizes with the inhibition of its expression using U1i.** The luciferase activity in cells transfected with a control plasmid has been calculated with one or half a dose of a plasmid expressing the shRNA against the luciferase target sequence 1546 (shRNAαLuc1546 and shRNAαLuc1546/2, respectively), with one or half a dose of a plasmid expressing a U1i against luciferase (U1iαLuc and U1iαLuc/2, respectively) and with half a dose of shRNAαLuc1546 and half a dose of U1iαLuc (shRNAαLuc1546/2 + U1iαLuc/2). The inhibition factor, which is the result of dividing the luciferase activity of the control (non-inhibited) cells by the luciferase activity obtained in the rest of the cases, is represented. The error bars represent the standard deviation of 9 independent measurements.

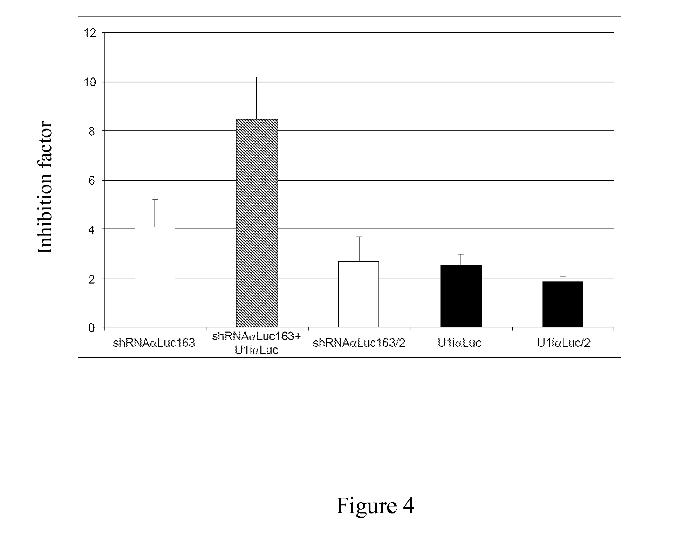

**Figure 4****. The inhibition of the expression of a reporter gene using medium shRNAs synergizes with the inhibition of its expression using U1i.** The luciferase activity of cells transfected with a control plasmid has been calculated with a one or half a dose of a plasmid expressing the shRNA against the luciferase target sequence 163 (shRNAαLuc163 and shRNAαLuc163/2, respectively), with a one or half a dose of a plasmid expressing a U1i against luciferase (U1iαLuc and U1iαLuc/2, respectively) and with half a dose of shRNAαLuc163 and half a dose of U1iαLuc (shRNAαLuc163/2 + U1iαLuc/2). The inhibition factor, which is the result of dividing the luciferase activity of the control (non-inhibited) cells by the luciferase activity obtained in the rest of the cases, is represented. The error bars represent the standard deviation of 9 independent measurements.

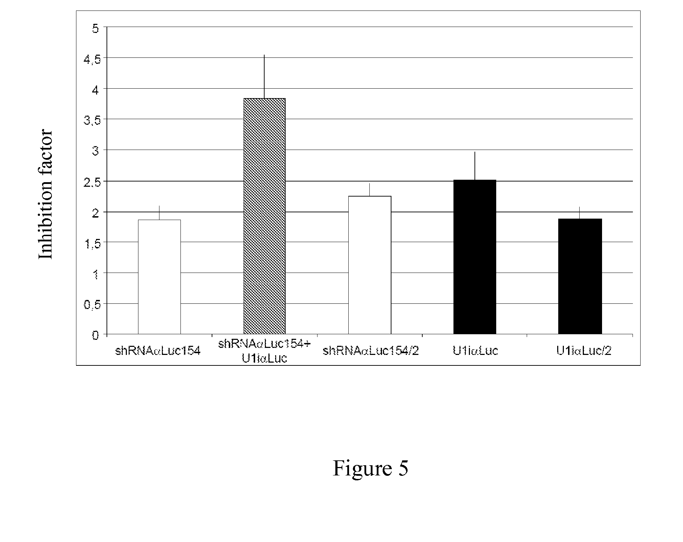

**Figure 5****. The inhibition of the expression of a reporter gene using weak shRNAs synergizes with the inhibition of its expression using U1i.** The luciferase activity of cells transfected with a control plasmid has been calculated with a one or half a dose of a plasmid expressing the shRNA against the luciferase target sequence 154 (shRNAαLuc154 and shRNAαLuc154/2, respectively), with a one or half a dose of a plasmid expressing a U1i against luciferase (U1iαLuc and U1iαLuc/2, respectively) and with half a dose of shRNAαLuc154 and half a dose of U1iαLuc (shRNAαLuc154/2 + U1iαLuc/2). The inhibition factor, which is the result of dividing the luciferase activity of the control (non-inhibited) cells by the luciferase activity obtained in the rest of the cases, is represented. The error bars represent the standard deviation of 9 independent measurements.

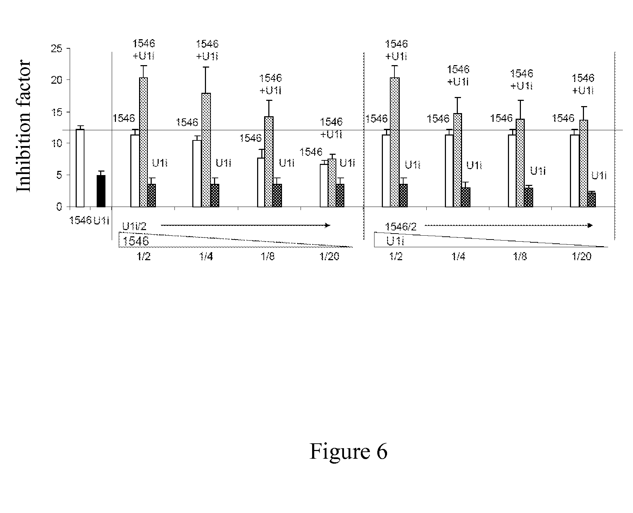

**Figure 6****. Analysis of the robustness of the synergy using strong shRNAs.** The luciferase activity of cells transfected with a control plasmid has been calculated with decreasing doses (1, ½, ¼, 1/8, and 1/20) of a plasmid expressing the shRNA against the luciferase target sequence 1546 (1546), with decreasing doses (1, ½, ¼, 1/8, and 1/20) of a plasmid expressing the U1i against luciferase (U1iαLuc), with half a dose of U1iαLuc and decreasing doses of shRNAαLuc1546 (½, ¼, 1/8, and 1/20) and with half a dose of shRNAαLuc1546 and decreasing doses of U1iαLuc (½, ¼, 1/8, and 1/20). The inhibition factor, which is the result of dividing the luciferase activity of the control (non-inhibited) cells by the luciferase activity obtained in the rest of the cases, is represented. The error bars represent the standard deviation of 9 independent measurements.

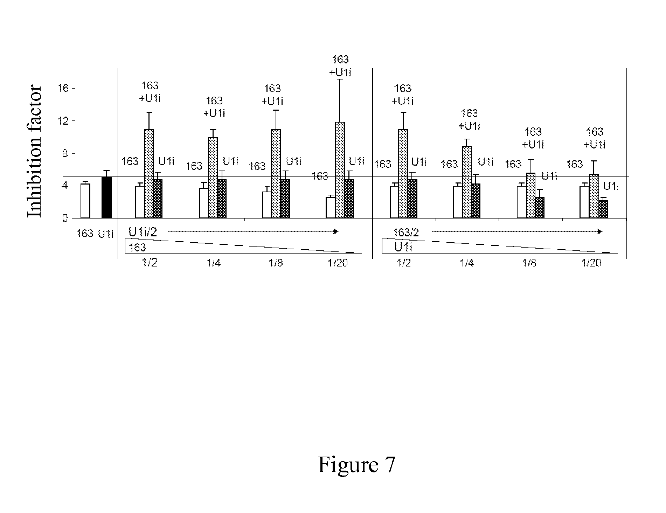

**Figure 7****. Analysis of the robustness of the synergy using medium shRNAs.** The luciferase activity of cells transfected with a control plasmid has been calculated with decreasing doses (1, ½, ¼, 1/8, and 1/20) of a plasmid expressing the shRNA against the luciferase target sequence 163 (163), with decreasing doses (1, ½, ¼, 1/8, and 1/20) of a plasmid expressing the U1i against luciferase (U1iαLuc), with half a dose of U1iαLuc and decreasing doses of shRNAαLuc163 (½, ¼, 1/8, and 1/20) and with half a dose of shRNAαLuc163 and decreasing doses of U1iαLuc (½, ¼, 1/8, and 1/20). The inhibition factor, which is the result of dividing the luciferase activity of the control (non-inhibited) cells by the luciferase activity obtained in the rest of the cases is represented. The error bars represent the standard deviation of 9 independent measurements.

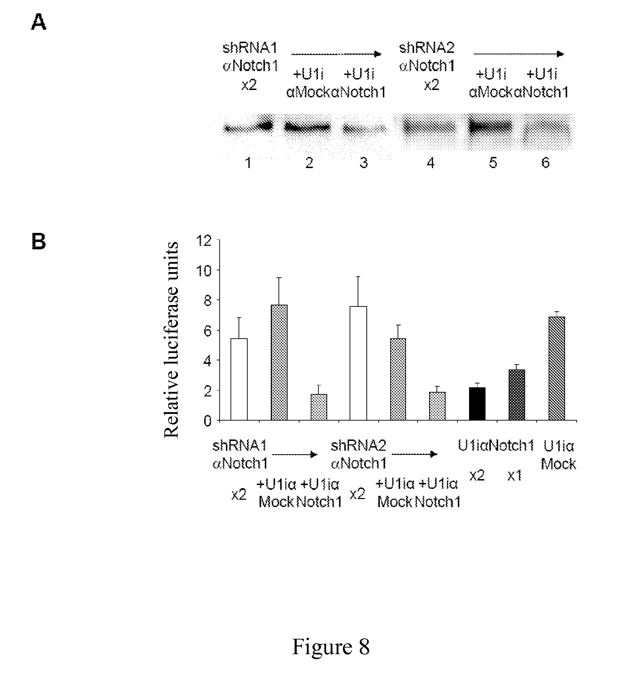

**Figure 8****. The inhibition of an endogenous gene using shRNAs synergizes with the inhibition of its expression using U1i. A.** HeLa cells have been transfected with two doses of plasmids expressing an shRNA against Notch1 target sequence 1 or 2 (shRNA1αNotch1 x2 and shRNA2αNotch1 x2, respectively), or a mixture of one dose of the plasmids expressing the shRNAs and one dose of plasmids expressing U1iαMock or U1iαNotchl. The cell extracts have been collected 48 hours after the transfection and the amount of Notch1 has been evaluated by means of Western blot. **B**. Cells, like those described in A, or transfected with U1iαMock or with one or two doses of U1iαNotch1, have been co-transfected with a plasmid expressing luciferase under a NF-κB-dependent promoter. The luciferase activity has been represented in relative units. The error bars represent the standard deviation of 9 independent measurements.

### DETAILED DESCRIPTION

The authors of the present invention have shown that, surprisingly, the combined use of U1 snRNAs specific for a certain target gene and gene expression-silencing agents directed to the same target gene results in an inhibition of the expression of the target gene greater than that observed when each of the agents are used separately.

Without wishing to be bound by any theory, it is believed that the synergic effect is due to the fact that the two components act at different points in the processing of the pre-mRNAs. Thus, while the modified U1s act in the nucleus by inhibiting the polyadenylation of the pre-mRNA and, consequently, they prevent its export to cytosol, the gene expression-silencing agents would act in the cytosol on the mRNAs that have managed to mature in the presence of the U1 inhibitor, causing the degradation thereof or the inhibition of the translation thereof. The modified U1s capable of inhibiting the polyadenylation of the target pre-RNAms are hereinafter referred to as U1is.

Thus, in a first aspect, the invention relates to a composition formed by one or several containers or a kit-of-parts which comprises:
(i) a first component comprising at least an U1 snRNA or a polynucleotide encoding a U1 snRNA, wherein said U1 snRNA is modified in its binding sequence to the GU consensus sequence of the 5' end of the intron such that it binds specifically to a pre-selected region in the 3' terminal exon of a target pre-mRNA and which is capable of inhibiting the maturation of the target pre-mRNA, and
(ii) a second component comprising at least one gene expression-silencing agent targeted specifically at a pre-selected region of the mRNA resulting from the processing of the pre-mRNA which is the target of the U1 snRNA defined in (i) and which is capable of causing the silencing of said target mRNA.

The compositions of the invention therefore comprise two components that can be found together in one and the same container or physically separated. Moreover, the invention also relates to a composition comprising components (i) and (ii) as defined above.

### The first component of the composition of the invention

The first component of the composition or kit of the invention is a polynucleotide encoding a U1 snRNA modified in its binding sequence to the GU consensus sequence of the 5' end of the intron such that it binds specifically to a pre-selected region in the 3' terminal exon of a target pre-mRNA and prevents the processing of the 3' end of said pre-mRNA, the processing of the 3' end being understood as any of the steps necessary for the formation of the polyadenylated mRNA from a non-polyadenylated pre-mRNA (processing of the consensus polyadenylation site and the addition of the poly (A)+ tail). These modified U1 snRNAs are incorporated in the corresponding snRNP to give rise to modified snRNPs that, instead of binding to the pre-mRNA in the intron processing site, bind to the pre-mRNA in a certain region thereof according to the sequence specificity of the modified region in the U1 snRNA. This interaction causes a stop in the polyadenylation machinery that results in the sequestering of the pre-mRNA in the nucleus, a reduction of the maturation of the pre-mRNA and, in turn, a reduction in the production of the encoded protein. In view of the capacity of modified U1 snRNAs to cause the inhibition of the expression of the target pre-mRNA, these modified U1s are known as U1i. The effect of the U1 is then a reduction or an inhibition of gene expression

The "binding sequence to the GU consensus sequence of the 5' end of the intron" of the modified U1 snRNA is understood as the region formed by the 5' end of the U1i which interacts by means of base pairing with the so-called U1 site or 5' processing site (5'-splice site) that appears in the pre-mRNAs in the 5' region of the introns, characterized by the AG/GURAGU consensus sequence (wherein R is a purine and/ indicates the exon/intron boundary) and allows the processing machinery of the pre-mRNAs to locate the exon-intron limit. This region corresponds to the nucleotides in positions 1 to 11 of the 5' end of the U1 snRNA sequence (Zhuang, Y and Weiner, A.M: 1986, Cell, 46:827-835).

The "modification" of the consensus sequence of binding to the U1 site in the pre-mRNA is understood as that the consensus sequence contains a number of mutations resulting in a substantial loss in the capacity of U1 snRNA to bind to the U1 site in the pre-mRNA while at the same time said U1 snRNA acquires a new complementarity allowing it to form a duplex by means of base pairing with a pre-selected region in a target pre-mRNA. The degree of complementarity between the sequence of binding to the U1 site modified according to the present invention and the pre-selected region in the target pre-mRNA can vary between 3 and 16 nucleotides, preferably between 8 and 16 nucleotides and, more preferably, between 10 and 11 nucleotides. However, the maximum efficiency of the inhibition of the expression of the target gene is observed when the 10-11 nucleotides forming the U1 site have been modified to make them complementary to the pre-selected site in the target pre-mRNA (see Figure 1). Therefore, the U1i will preferably be modified in all those nucleotides in positions 1 to 11 or 2 to 11, such that said region will be completely complementary with 11 or 10 consecutive nucleotides in the pre-selected site of the target pre-mRNA. A person skilled in the art will appreciate that, in order to generate the U1i, it will only be necessary to modify those nucleotides in the native U1 which are different from those appearing in the target sequence.

A person skilled in the art will appreciate that the U1 snRNA sequences of different organisms are known. Therefore, once the sequence of a certain U1 snRNA is known, it is possible to determine if it corresponds to a native U1 snRNA or has been modified in one or more nucleotides of the region interacting with the 5' end of the intron.

The region in the target mRNA that is pre-selected for its incorporation as a complementary sequence in the 5' region of the modified U1 is located in the terminal exon of the corresponding pre-mRNA. The distance of the target sequence to the polyadenylation site is not particularly relevant as has been shown by Fortes et al. (*supra*) in which it was shown that modified U1s s targeted at the terminal exon of RNA of a reporter gene were capable of causing the degradation of said RNA, even when the target sequence was located at more than 1000 nucleotides from the polyadenylation sequence.

Given that the minimum optimum length of the target sequence is 10 nucleotides, the sites of binding for a sequence of said length will appear randomly once every 10⁶ nucleotides, which implies that they will appear about 3000 times in the human genome. However, given that exons form only about 2% of the human genome and that the inhibition mediated by snRNP with modified U1s s requires pairing to non-structured regions in the terminal exons of the transcripts containing the polyadenylation signals (AAUAAA), the specificity of the inhibition is much greater. The target sites that appear the least number of times in the 3' terminal exons of the organism from which the cells in which the silencing will be carried out come will preferably be chosen.

The frequency of appearance of the possible targets may be determined by sequence comparison. Typically, for sequence comparison, one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 19 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith and Waterman, (Adv. Appl. Math., 1981, 2:482), by the homology alignment algorithm of Needleman and Wunsch, (J. Mol. Biol., 1970, 48:443), by the search for similarity method of Pearson and Lipman, (Proc. Natl Acad. Sci. USA, 1988, 85:2444), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Current Protocols in Molecular Biology (Ausubel et ah, eds. 1995 supplement)).

A preferred example of algorithm that is suitable for determining percent sequence identity and sequence similarity are the BLAST and BLAST 2.0 algorithms, which are described in Altschul et al, (Nucl. Acids Res., 1977, 25:3389-3402) and Altschul et al, (J. MoI. Biol., 1990, 215:403-410 ), respectively. BLAST and BLAST 2.0 are used, with the parameters described herein, to determine percent sequence identity. Software for performing BLAST analyses is publicly available through the National Center for Biotechnology Information. This algorithm involves first identifying high scoring sequence pairs (HSPs) by identifying short words of length W in the query sequence, which either match or satisfy some positive- valued threshold score T when aligned with a word of the same length in a database sequence. T is referred to as the neighborhood word score threshold (Altschul et al, supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always 0) and N (penalty score for mismatching residues; always 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff, Proc. Natl. Acad. Sd. USA, 1989, 89:10915) alignments (B) of 50, expectation (E) of 10, M=5, N=-4, and a comparison of both strands.

Preferably, the modified U1 snRNAs used in the compositions of the invention are selected so that its binding sequence to the GU consensus sequence of the 5' end of the intron, which has been modified according to the sequence of the target pre-mRNA is identical or substantially identical to the target sites. The terms "identical" or percent "identity," in the context of two or more nucleic acids or polypeptide sequences, refer to two or more sequences or subsequences that are the same (identical) or have a specified percentage of amino acid residues or nucleotides that are the same (i.e., at least 70% identity, preferably 70%, 75%, 80%, 85%, 90%, 91%, 92%, 93%, 94%, 95%, 96%, 97%, 98%, 99%, or higher identity over a specified region of a target sequence, when compared and aligned for maximum correspondence over a comparison window or designated region) as measured using a BLAST or BLAST 2.0 sequence comparison algorithms with default parameters described above, or by manual alignment and visual inspection (see, e.g., NCBI web site or the like). Such sequences are then said to be "substantially identical."

In the event that a family of proteins with related sequences is to be silenced or in the event that the exact particular sequence of the mRNA which is to be silenced is not known (for example, in the case of viral proteins showing different serotypes), it is possible to design U1 inhibitors allowing the simultaneous silencing of several proteins. To that end, it is necessary to identify conserved sequences within the nucleotide sequences of the group of proteins to be silenced. This can be carried out by means of multiple alignment of the sequences using algorithms widely known to a person skilled in the art (for example the ClustalW algorithm, described by Chenna et al., 2003, Nucleic Acids Res., 31:3497-3500). Once the conserved sequence regions among the different genes have been identified, a consensus region will be designed which can be used as a target for designing the specific U1 snRNAs for a family of proteins. The design of the U1 inhibitors the 5' sequence of which is targeted at the conserved sequences among the different members of the family will result in the simultaneous silencing of several members of the family. In the event that the members of the family show a high degree of identity and there are multiple conserved regions that can be chosen as a target for designing the U1 inhibitor, those appearing the least number of times in the 3' terminal exon of the RNAs of the organism in which the silencing is carried out, determined as indicated in the previous paragraph, will be chosen.

The polynucleotide encoding the U1 snRNA according to the invention can be operatively coupled to a promoter allowing the transcription of said polynucleotide in the cell in which the U1i is to be expressed. Promoters suitable for carrying out the present invention include, without necessarily being limited to, constitutive promoters such as the U1 promoter or promoters derived from eukaryotic virus genomes such as the polyomavirus, adenovirus, SV40, CMV, the avian sarcoma virus, the hepatitis B virus, the metallothionein gene promoter, the thymidine kinase gene promoter of the herpes simplex virus, LTR regions of retroviruses, the immunoglobulin gene promoter, the actin gene promoter, the EF-1 alpha gene promoter, as well as inducible promoters in which the expression of the gene depends on the addition of a molecule or of an exogenous signal, such as the tetracycline system, the NF-kappa B/UV light system, the Cre/Lox system and the heat-shock gene promoter, the regulatable RNA polymerase II promoters described in WO/2006/135436, as well as tissue-specific promoters (for example, the PSA promoter described in WO2006012221). In a preferred embodiment, the promoters are the RNA polymerase II promoters acting in a constitutive manner.

It is also possible to use tissue-specific promoters in the event that the gene the expression of which is to be silenced is specifically expressed in a tissue:
- a specific stomach promoter, such as the H/K-ATPase beta subunit promoter, the K19 promoter, the metallothionein promoter, the TFF1 promoter, the TFF2 promoter, the FOXa/HNF2 gamma promoter,
- a specific pancreas promoter, such as the elastase promoter, the Pdx-1 promoter, the insulin promoter or the phosphoglycerate kinase promoter,
- a specific lung promoter such as the Clara cell secretory protein promoter, the surfactant protein C promoter,
- a specific breast promoter, such as the mouse mammary tumour virus promoter or the whey acidic protein promoter,
- a specific skin promoter, including the keratin promoter or the K14 promoter,
- a specific oesophagus promoter, such as EBV promoter 12,
- a specific liver promoter, such as the major urinary protein promoter or the albumin promoter,
- a specific colon promoter, such as the villin promoter or the FABP-TS4 promoter,
- a specific prostrate promoter, such as the cryptidin-2 promoter, the prostate-specific antigen promoter, the C(3)1 promoter, the promoter of the protein secreted by the prostate with 94 amino acids (PSP94) or the probasin promoter,
- a specific kidney promoter, such as the uromodulin promoter, the Tamm-Horsfall protein promoter or the type 1 gamma-glutamyl transpeptidase promoter,
- a specific bladder promoter, such as the uroplakin promoter or the urohingin promoter,
- a specific uterus promoter, such as the uteroglobin promoter.

Additionally, any tissue-specific promoter described in the TiProD tissue-specific promoter database as described by Chen, X et al., (Nucleic Acids Res., 2006, 34, Database Issue) can be used in the context of the present invention.

### Second component of the composition of the invention

The second component of the composition according to the invention is a gene expression-silencing agent or a combination of said gene expression-silencing agents specific for the mRNA resulting from the processing of the target pre-mRNA against which the U1i forming part of the first component of the invention and which is capable of causing the silencing of the expression of said mRNA was targeted.

"Gene expression-silencing agents" are understood as those compounds causing the degradation of a target mRNA or inhibiting its translation by means of a so-called RNA interference process (RNAi). In this process, a double-strand RNA (dsRNA) is capable of causing the silencing of gene expression by means of converting said RNA into siRNA by means of an RNase type III (Dicer). One of the siRNA strands is incorporated into the ribonucleoprotein complex referred to as the RNA-induced silencing complex (RISC). The RISC complex uses this single strand of RNA to identify mRNA molecules that are at least partially complementary to the RNA strand of the siRNA incorporated in the RISC that are degraded or undergo an inhibition in their translation. Thus, the siRNA strand that is incorporated into the RISC is known as a guide strand or antisense strand. The other strand, which is known as a transient strand or sense strand, is eliminated from the siRNA and is partly homologous to the target mRNA. The degradation of a target mRNA by means of the RISC complex results in a reduction in the expression levels of said mRNA and of the corresponding protein encoded thereby. Furthermore, RISC can also cause the reduction in the expression by means of the inhibition of the translation of the target mRNA.

An agent "capable of causing the silencing of the expression of said mRNA" is understood, in the context of this invention, as an agent that, when it is inside a cell, is capable of encoding for an agent capable of or which is itself capable of binding to a target mRNA containing the pre-selected sequence and causing the degradation thereof.

Silencing agents inducing the RNAi response against a target mRNA and which could be incorporated into the second component of the invention include:
(i) siRNA
(ii) shRNA
(iii) miRNA
(iv) a polynucleotide encoding an agent according to (i), (ii) or (iii) and
(v) a combination of one or several agents according to (i) to (iv)
wherein the agents (i), (ii) and (iii) are based on RNA and the component (iv) is based on DNA.

### (i) siRNA

siRNAs are agents capable of inhibiting the expression of a target gene by means of RNA interference. A siRNA can be chemically synthesised or can be obtained through *in vitro* transcription. siRNAs typically consist of a double RNA strand with a length between 15 and 40 nucleotides and can contain a 3' and/or 5' overhanging region with 1 to 6 nucleotides. The length of the overhanging region is independent of the total length of the siRNA molecule. siRNAs act by means of the degradation or the post-transcriptional silencing of the target messenger. The siRNAs of the invention are substantially homologous with a pre-selected region of the target mRNA. "Substantially homologous" is understood as that they have a sequence which is sufficiently complementary or similar to the target mRNA such that the siRNA is capable of causing the degradation thereof by RNA interference. Substantially homologous may mean substantial identity as defined above. The siRNAs suitable for causing said interference include siRNAs formed by RNA, as well as siRNAs containing different chemical modifications such as:
- siRNAs in which the bonds between the nucleotides are different from those that occur in nature, such as phosphorothioate bonds,
- conjugates of the siRNA strand with a functional reagent, such as a fluorophore,
- Modifications of the ends of the siRNA strands, particularly the 3' end by means of the modification with different functional groups of the hydroxyl in position 2',
- Nucleotides with modified sugars such as O-alkylated moieties in position 2' such as 2'-O-methylribose p 2'-O-fluororibose,
- Nucleotides with modified bases like halogenated bases (for example 5-bromouracil and 5- iodouracil), alkylated bases (for example 7-methylguanosine).

The siRNAs of the invention can be obtained using a series of techniques well-known to a person skilled in the art. For example, the siRNA can be chemically synthesised starting from ribonucleosides protected with phosphoramidite groups in a conventional DNA/RNA synthesizer.

### (ii) shRNA

In another method, the second component of the invention is shRNA (short hairpin RNA). shRNA is understood, in the context of this invention, as an RNA molecule formed by two anti-parallel strands connected by a hairpin region and wherein the sequence of one of the anti-parallel strands is complementary to a pre-selected region in the target mRNA. The shRNAs are formed by a short antisense sequence (with 19 to 25 nucleotides), followed by a loop of 5 to 9 nucleotides followed by the sense strand. shRNAs can be chemically synthesized from ribonucleosides protected with phosphoramidite groups in a conventional DNA/RNA synthesizer or they can be obtained from a polynucleotide by means of *in vitro* transcription. shRNAs are processed inside the cell by the RNase Dicer that eliminates the hairpin region giving rise to siRNAs as has been previously described. shRNAs can also contain distinct chemical modifications as has been previously described in the case of siRNAs.

### (iii) miRNA

miRNAs or microRNAs are small RNA molecules that appear naturally in the cell and are responsible for controlling the specificity of gene expression by regulating the degradation and translation of mRNA. miRNAs are single-strand RNA molecules with 22-nucleotides that are synthesized like large primary transcripts (pri-miRNA) that are processed in the nucleus by the Drosha RNase type III to give rise to "hairpin" precursors of 60 bases pairs (pre-miRNA). These molecules are transported to the cytoplasm and processed by a second nuclease (Dicer) to give rise to the mature forms (miRNA). The miRNAs are incorporated into the ribonucleoprotein RISC complex, where they act causing the degradation of target mRNA by means of pairing between the mature mRNA-RISC complex and a homologous region of the target mRNA, in particular between the so-called "seed" region of the miRNA strand (nucleotides 2 to 7 of the 5' end), resulting in the degradation of the mRNA and/or translational attenuation. Although miRNAs are endogenous molecules that act by regulating the half-life of mRNA cells, it has been shown that the structure of an endogenous mRNA (for example, miR-30) can be altered to include sequences encoding siRNAs and that said modified miRNAs can be used to cause the degradation of the endogenous target genes (WO03093441). It has also been shown that the siRNA sequences incorporated in the mir-26a stem region work as RNAi effectors causing the degradation of homologous mRNAs (McManus, M.T. et al., 2002, RNA, 8:842-850). In both cases the nucleotide sequence introduced into the miRNA stem showed a 100% identity with the target gene.

The miRNAs suitable for their use in the compositions of the invention consist of 19 to about 24 nucleotides, preferably 21 or 22 nucleotides. The miRNAs can be designed such that they hybridize to an RNA transcript with a high degree of specificity. The miRNA is preferably designed such that it shows a 100% identity or which shows a substantial identity (i.e. allowing at least 1, at least 2, at least 3 or more mismatches) with the target mRNA given that only one non-complementary nucleotide can, depending on its position in the miRNA strand, reduce the inhibition levels. The miRNAs can be designed such that they target the non-translated 5' region, the encoding region or the 3' region of the target mRNA.

The miRNAs are derived from the processing of pre-miRNAs comprising a stem-loop region. This structure can be designed such that it is recognised by a ribonuclease, preferably by a ribonuclease type III like a Dicer, resulting in the formation of the mature miRNA. The stem-loop structures can have 40 to 100 nucleotides, preferably between 50 and 70 nucleotides and, more preferably, between 20 and 30 nucleotides. The stem can comprise a perfectly complementary duplex or can contain additional non-complementary zones in at least one of the strands forming the duplex. Preferably, said non-complementary zones are not very abundant (for example, 1, 2 or 3) and consist of 3 nucleotides or less in size. The loop terminal can comprise 4 or more nucleotides (preferably not more than 25). The loop is preferably of 6 to 15 nucleotides in size.

The efficient processing and functioning of the miRNA is typically only possible when said miRNA has certain structural requirements, such as those described by Zeng et al. (RNA, 2003, 9:112-123). The miRNAs of the invention are preferably based on the mirR-30 structure in which the stem region has been replaced with target sequences of pre-selected mRNAs. The presence of miR-30 in the loop region, although desirable, is not absolutely necessary since it can tolerate certain variations such that the loop region has more than 70%, preferably more than 79%, even more preferably more than 86%, and even more preferably, more than 93% identity with respect to the loop sequence that appears in miR-30. Determination of the percent identity can be determined using any of the methods mentioned above.

The miRNAs suitable for carrying out the present invention correspond to those based on the endogenous miR-30 miRNA, as has been described in WO03093441 or based on the endogenous miRNAs as has been described in WO03029459.

### (iv) Polynucleotides encoding siRNA, shRNA or miRNA.

The second component of the invention can be provided as a polynucleotide the transcription of which gives rise to the previously described siRNAs, shRNAs and/or miRNAs. In the case of polynucleotides encoding a shRNA or a miRNA, they comprise a single promoter region regulating the transcription of a sequence comprising the sense and antisense strands of the shRNAs and miRNAs connected by a hairpin or by a stem-loop region. In principle, any promoter can be used for the expression of the shRNAs and miRNAs provided that said promoters are compatible with the cells in which the siRNAs are to be expressed. Thus, the promoters suitable for carrying out this invention include those mentioned previously for the expression of U1i. In a preferred embodiment, the promoters are RNA polymerase III promoters that act constitutively. The RNA polymerase III promoters appear in a limited number of genes such as 5S RNA, tRNA, RNA 7SL and snRNA U6.

In addition, the polynucleotides encoding siRNAs comprise two transcriptional units, each formed by a promoter regulating the transcription of one of the strands formed in siRNA (sense and antisense). The polynucleotides encoding siRNAs can contain convergent or divergent transcriptional units. In the divergent transcription polynucleotides, the transcriptional units encoding each of the DNA strands forming the siRNA are located in tandem in the polynucleotide such that the transcription of each DNA strand depends on its own promoter, which can be the same or different (Wang, J. et al., 2003, Proc. Natl. Acad. Sci. USA, 100:5103-5106 and Lee, N.S., et al., 2002, Nat. Biotechnol., 20:500-505). In the convergent transcription polynucleotides, the DNA regions giving rise to the siRNAs form the sense and antisense strands of a DNA region that is flanked by two inverted promoters. After the transcription of the sense and antisense RNA strands, they will form the hybrid corresponding to the functional siRNA. Promoter combinations suitable for the polynucleotides comprising inverted transcriptional units include 2 U6 promoters (Tran, N. et al., 2003, BMC Biotechnol., 3:21), a mouse U6 promoter and a human H1 promoter (Zheng, L., et al., 2004, Proc. Natl. Acad. Sci. USA, 101:135-140 and WO2005026322) and a human U6 promoter and a mouse H1 promoter (Kaykas, A. & Moon, R., 2004, BMC Cell Biol., 5:16). In a preferred embodiment, the sense and antisense siRNA strands are regulated by different promoters. In an even more preferred embodiment, both transcriptional units are oriented in a convergent manner. In a preferred embodiment, the RNA polymerase type III promoters are the promoters of the H1 and U6 genes of human and murine origin. In an even more preferred embodiment, the promoters are 2 U6 promoters of human or murine origin, a mouse U6 promoter and a human H1 promoter or a human U6 promoter and a mouse H1 promoter.

### (v) Combinations of one or more siRNAs, shRNAs, miRNAs and/or of the polynucleotides encoding them

The second component of the invention can be formed by one or more of the components indicated in (i), (ii), (iii) and (iv). Thus, the invention contemplates the combined use of one or more siRNAs, one or more shRNAs, one or more miRNAs and/or one or several of the polynucleotides encoding said polynucleotides. The different silencing agents can be targeted against different regions of the one and the same mRNA.

In a preferred embodiment, component (ii) of the composition or kit of parts of the invention is a polynucleotide comprising a sequence encoding at least one shRNA.

### Vectors and gene constructs of the invention

The polynucleotides encoding the first component of the invention, as well as the polynucleotides encoding siRNAs, miRNAs and/or shRNAs and forming the second component of the invention can be found isolated as such or forming part of vectors allowing the propagation of said polynucleotides in suitable host cells. Vectors suitable for the insertion of said polynucleotides are vectors derived from expression vectors in prokaryotes such as pUC18, pUC19, Bluescript and the derivatives thereof, mp18, mp19, pBR322, pMB9, Co1E1, pCR1, RP4, phages and "shuttle" vectors such as pSA3 and pAT28, expression vectors in yeasts such as vectors of the type of 2 micron plasmids, integration plasmids, YEP vectors, centromere plasmids and the like, expression vectors in insect cells such as vectors of the pAC series and of the pVL, expression vectors in plants such as pIBI, pEarleyGate, pAVA, pCAMBIA, pGSA, pGWB, pMDC, pMY, pORE series and the like, and expression vectors in eukaryotic cells, including baculovirus suitable for transfecting insect cells using any commercially available baculovirus system. The vectors for eukaryotic cells include preferrably viral vectors (adenoviruses, viruses associated to adenoviruses such as retroviruses and, particularly, lentiviruses) as well as non-viral vectors such as pSilencer 4.1-CMV (Ambion), pcDNA3, pcDNA3.1/hyg, pHMCV/Zeo, pCR3.1, pEFI/His, pIND/GS, pRc/HCMV2, pSV40/Zeo2, pTRACER-HCMV, pUB6/V5-His, pVAX1 pZeoSV2, pCI, pSVL and PKSV-10, pBPV-1, pML2d and pTDT1.

In a preferred embodiment, the polynucleotide encoding the U1i RNA and the polynucleotide encoding the siRNA, shRNA or miRNA form part of the same vector. In another preferred embodiment, the polynucleotide encoding the U1i RNA and the polynucleotide encoding the siRNA, shRNA or miRNA form part of a single vector.

The vectors preferably comprise a reporter or marker gene which allows identifying those cells that have been incorporated the vector after having been put in contact with it. Useful reporter genes in the context of the present invention include lacZ, luciferase, thymidine kinase, GFP and on the like. Useful marker genes in the context of this invention include, for example, the neomycin resistance gene, conferring resistance to the aminoglycoside G418; the hygromycin phosphotransferase gene, conferring resistance to hygromycin; the ODC gene, conferring resistance to the inhibitor of the ornithine decarboxylase (2-(difluoromethyl)-DL-ornithine (DFMO); the dihydrofolate reductase gene, conferring resistance to methotrexate; the puromycin-N-acetyl transferase gene, conferring resistance to puromycin; the ble gene, conferring resistance to zeocin; the adenosine deaminase gene, conferring resistance to 9-beta-D-xylofuranose adenine; the cytosine deaminase gene, allowing the cells to grow in the presence of *N*-(phosphonacetyl)-L-aspartate; thymidine kinase, allowing the cells to grow in the presence of aminopterin; the xanthine-guanine phosphoribosyltransferase gene, allowing the cells to grow in the presence of xanthine and the absence of guanine; the trpB gene of E. coli, allowing the cells to grow in the presence of indol instead of tryptophan; the hisD gene of *E*. *coli*, allowing the cells to use histidinol instead of histidine. The selection gene is incorporated into a plasmid that can additionally include a promoter suitable for the expression of said gene in eukaryotic cells (for example, the CMV or SV40 promoters), an optimized translation initiation site (for example, a site following the so-called Kozak's rules or an IRES), a polyadenylation site such as, for example, the SV40 polyadenylation or phosphoglycerate kinase site, introns such as, for example, the beta-globulin gene intron. Alternatively, it is possible to use a combination of both the reporter gene and the marker gene simultaneously in the same vector.

In a preferred embodiment, the vectors are viral vectors. In an even more preferred embodiment, the vector used for the expression of the first or the second component of the invention (provided that it is based on DNA) is a viral vector that is selected from the group of adenoviruses, adeno-associated viruses, retroviruses, lentiviruses, herpes viruses, SV40 and alphaviruses.

The region of the target mRNA which is taken as a basis for designing the siRNAs is not limiting and can contain a region of the encoding sequence (between the start codon and the stop codon) or, alternatively, it may contain sequences of the non-translated 5' or 3' region. Preferably, it will have a length between 21 and 50 nucleotides.

The criteria for designing siRNAs, shRNAs and miRNAs are widely known to the person skilled in the art (for example, see Birmingham, A. et al., 2007, Nature Protocols, 2:2068-2078, Ladunga, I., 2006, Nucleic Acids Res. 35:433-440 and Martineau, H., Pyrah, I., 2007, Toxicol. Pathol., 35:327-336 and Pei and Tuschl, 2006, Nature Methods, 3:670-676).

The criteria for designing U1is suitable for inhibiting the expression of target genes have been explained above (see Fortes, P. et al., Proc. Natl. Acad. Sci. USA, 2003, 100:8264-8269 and Sajic, R. et al., 2006, Nucleic Acids Res., 35:247-255). Preferably, the region of the target pre-mRNA that is taken as a basis for designing the U1is comprises only the 3' terminal region as has been shown by Fortes et al. (*supra*).

In a preferred embodiment, component (i) of the kit of parts or composition of the invention comprises several U1 snRNAs or several polynucleotides encoding different U1is, all of them being targeted to different regions of one and the same target mRNA. In this way, a synergic inhibitor effect of the U1is can be obtained, as has been shown by Fortes et al. (*supra*) and Sajic et al. (*supra*).

In another preferred embodiment, component (ii) of the kit of parts or composition of the invention comprises several gene expression-silencing agent targeted specifically at different pre-selected region of the mRNA resulting from the processing of the pre-mRNA which is the target of the U1 snRNA as defined in (i) and which are capable of causing the *in vivo* silencing of said target mRNA.

In a still more preferred embodiment, component (i) of the kit of parts or composition of the invention comprises several U1 snRNAs or several polynucleotides encoding U1 snRNAs modified in their binding sequences to the GU consensus sequence of the 5' end of the intron so that they bind specifically to different pre-selected regions of the same target pre-mRNA and the component (ii) of the kit of parts or composition comprises several gene expression-silencing agent targeted specifically at different pre-selected region of the mRNA resulting from the processing of the pre-mRNA which is the target of the U1 snRNA as defined in (i) and which are capable of causing the *in vivo* silencing of said target mRNA.

The compositions of the invention can contain both components in a single container or the two components can be physically separated in several containers, in which case, the composition is known as "kit of the invention". A "kit" is understood, in the context of this invention, as a product containing the different components of the composition of the invention packaged to permit the transport and storage thereof. Materials suitable for the packaging of the kit components include glass, plastic (polyethylene, polypropylene, polycarbonate and the like), bottles, vials, paper, envelopes and the like. Additionally, the kits of the invention can contain instructions for the simultaneous, sequential or separate administration of the components in the kit. Said instructions can be in the form of printed material or in the form of an electronic support, capable of storing the instructions such that they can be read by a subject, such as electronic storage media (magnetic disks, tapes and similar), optical media (CD-ROM, DVD) and the like. Additionally or alternatively, the media can contain Internet addresses providing said instructions.

### Bifunctional polynucleotides of the invention

The authors of the present invention propose that when the second component of the invention is based on DNA and therefore consists of a sequence encoding a siRNA, an shRNA or an miRNA, it can be combined in a single polynucleotide with the sequence of first component of the invention and which encodes the U1i, which allows contacting the organism to be treated in with the two components necessary to obtain a simultaneous synergic effect by means of a single administration. Therefore, in another aspect, the invention relates to a polynucleotide (hereafter bifunctional polynucleotide of the invention) comprising
(i) a sequence encoding at least one U1 snRNA modified in its binding sequence to the GU consensus sequence of the 5' end of the intron such that it binds specifically to a pre-selected region of the 3' terminal exon of a target pre-mRNA and which is capable of inhibiting the processing of the target pre-mRNA, and
(ii) a sequence encoding a silencing agent targeted specifically at a pre-selected region of the mRNA resulting from the processing of the pre-mRNA which is the target of the U1 snRNA encoded by the polynucleotide defined in (i) and which is capable of causing the *in vivo* silencing of said target mRNA.

In a preferred embodiment, component (ii) of the bifunctional polynucleotide of the invention comprises the sense and antisense strands of at least one siRNA, encodes at least one pre-miRNA or encodes at least one shRNA.

In another aspect, the invention relates to a polynucleotide comprising
(i) a sequence encoding a U1 snRNA modified in its binding sequence to the GU consensus sequence of the 5' end of the intron such that it binds specifically to a pre-selected region of the 3' terminal exon of a target pre-mRNA and which is capable of inhibiting the maturation of the target pre-mRNA, and
(ii) a sequence encoding the sense and antisense strands of at least one siRNA, encoding at least one pre-miRNA, or encoding at least one shRNA wherein said siRNA, pre-miRNA and/or shRNA binds specifically to a pre-selected region of the mRNA resulting from the processing of the pre-mRNA which is the target of the U1 snRNA encoded by the polynucleotide defined in (i) and which is capable of causing the *in vivo* silencing of said target mRNA.

The bifunctional polynucleotides of the invention are usually coupled to regions regulating their expression which can be identical for the sequences encoding the U1i and for the sequences encoding the silencing agent or can be different. Suitable promoters for the expression of said polynucleotides are the same (constitutive, regulatable and tissue-specific) that were previously mentioned for the expression of U1is.

Likewise, the bifunctional polynucleotides of the invention can form part of an expression vector. Thus, in another aspect, the invention is related to expression vectors comprising the bifunctional polynucleotide of the invention. Suitable vectors for the cloning and for the propagation of the polynucleotides of the invention are substantially the same as those previously mentioned for the expression of the polynucleotides encoding the U1is or the polynucleotides encoding the siRNAs, the shRNAs and/or the miRNAs. Preferably, the expression vectors of the polynucleotides of the invention are viral vectors, even more preferably vectors selected from the group of adenoviruses, adeno-associated viruses, retroviruses, lentiviruses, herpes viruses, SV40 and alphaviruses.

In another aspect, the invention relates to a cell comprising a bifunctional polynucleotide of the invention or a vector comprising a bifunctional polynucleotide of the invention. Host cells suitable for the expression of the compositions of the invention include, without being limited to, the cells of mammals, plants, insects, fungi and bacteria. Bacterial cells include, without being limited to, Gram-positive bacterial cells such as species of the Bacillus, Streptomycin and Staphylococci genus and Gram-negative cells such as cells of the Escherichia and Pseudomonas genus. Fungal cells preferably include yeast cells such as *Saccharomyces, Pichia pastoris* and *Hansenula polymorpha.* Insect cells include, without limitation, Drosophila cells and Sf9 cells. Plant cells include, among others, crop plants such as cereals, medicinal, ornamental plants or bulb plants. Said cells are transfected with the bifunctional polynucleotides of the invention by means of known techniques such as Agrobacterium-mediated gene transfer, transformation of leaf discs, transformation induced by polyethylene glycol, electroporation, sonication, microinjection and biolistic transfer. Mammalian cells suitable for the insertion of the bifunctional polynucleotides of the invention include CHO (Chinese Hamster Ovary) cells, COS cells, BHK cells, HeLa cells, 911, AT1080, A549, 293 or PER.C6.

In another embodiment, the invention relates to a non-human transgenic animal comprising, integrated into its genome, a bifunctional polynucleotide according to the invention.

It will be appreciated that the method for obtaining a non-human animal can only be carried out when the bifunctional polynucleotide of the invention is inserted into the genome of a stem cell that can be differentiated in all cell types, including the germinal line cells. These are the embryonic cells (ECs) or embryonic germ cells (EGCs). Embryonic stem cells (ESCs) are derived from the internal cell mass of the embryo in the blastocyst stage before the implantation. Several types of mouse and human embryonic stem cells are known and have been established such as, for example, the ESC, TBV2, R1 and D3 cells in mice. These cells can be cultured *in vitro* in suitable culture conditions in a non-differentiated state and conserve the capacity to resume the normal *in vivo* development as the result of being combined with blastocysts and being inserted into the uterus of a pseudo-pregnant foster mother. Embryonic germ cells (EGCs) are derived from primordial germ cells (PGCs) cultured from the genital foetal ridge. Normally, the ESC and EGC cells of the invention that carry a modified GDNF locus are injected into a host blastocyst, i.e., the blastocele of the host blastocyst, or cultured together with the ovules of eight cells until the morula stage, that is, a zone-free morula, that the modified ESC cells are preferably incorporated into the internal cell mass of the embryo under development.

With the injection into the blastocyst, the transgenic litter is called chimeric, since some of its cells are derived from the host blastocyst and some derive from the modified ESC cells. The host embryos are transferred to substitute pseudo-pregnant females or intermediate hosts for continuous development. As a result, the procedure will provide chimeric animals the somatic and germinal line tissue of which comprises a mixture of cells derived from the ESC cells obtained by genetic engineering and the receptor blastocyst. Normally, stem cells and the blastocysts are obtained from animals that have different skin pigmentations such that the chimeric animals can have spots of different colours. Therefore, these chimeric animals are again crossed with their siblings until a transgenic animal in the germinal line is obtained, i.e., an animal in which the modified transgene is present in the germinal cells of the animal so that the characteristic can pass to the animal litter through reproduction. The transgenic animals in the germinal line can be identified, for example, by means of observing the litter to determine the presence of the characteristic or by means of the examination of the germinal cells of the transgenic animal to determine the presence of a transgene, incorporated in a way that it is consistent with the heritability. These animals are then crossed with other animals such that monozygotic animals are obtained for the desired characteristic, but which do not show mosaicism any more.

It must be understood that the non-human transgenic animals described herein can be produced by methods different from the ESC cell method described above, for example, by pronuclear injection of the targeted construct in the pronuclei of single-cell embryos or other gene targeting methods that are not based on a transfected ESC cell.

Any suitable non-human mammal can be used to produce the non-human transgenic mammal described herein. For example, a suitable mammal could be a rodent (e.g. mouse, rat, hamster, guinea pig, gerbil), a rabbit, a pig, a sheep, a goat, a cow, a cat, a dog, swine or a primate provided that the specific line or lines of the animal are selected to obtain good general health, good embryo performances, good pronuclear visibility in the embryo and good reproductive capacity. In a preferred embodiment, the animal is a mouse. Varieties such as the C57BL/6 or C57BL/6 x DBA/2 Fit, or FVB lines (commercially obtained from Charles River Labs., Boston, Mass., The Jackson Laboratory, Bar Harbor, ME or Taconic Labs.) are often used. The preferred varieties are the 129sv variety, as well as those with varieties that have H 2b, H-26 or H-2q haplotypes, such as C57BL/6 or DBA/1.

### Medical uses of the compositions of the invention

The authors of the present invention have shown that the compositions of the invention are capable of inhibiting the expression of a target gene in a synergic manner, i.e. more effectively than each of the components acting separately. This allows the use of the compositions in which the components are targeted against mRNAs of proteins involved in pathologies as a medical product. Thus, in another aspect, the invention relates to compositions of the invention and to the bifunctional nucleotides of the invention described above for their use in medicine or as a medicament. The person skilled in the art will appreciate that the compositions can be used for the treatment of all those diseases or conditions that result from the over-expression of a certain gene or which require a decrease in the expression of a given gene.

The skilled person will appreciate that the different possibilities exist for the administration of the composition of the invention. The two components of the composition may be administered in the form of separate molecules or the same molecule.

Moreover, both the first and the second component may be administered as the inhibitory molecules as such, i.e. the component (i) may be administered as the U1 snRNA form and component (ii) may be administered as a RNA-based silencing agent as such (siRNA, miRNA, shRNA. Alternatively, the first component may be provided as polynucleotide encoding the U1 snRNA and/or the second component may be DNA-based, i.e. it comprises a polynucleotide encoding the silencing agent. In the latter case, the polynucleotides may be administered as a single polynucleotide comprising both component (i) and component (ii) or as separate polynucleotides, either simultaneously, separately or sequentially.

Thus, the invention contemplates different combinations of administration regimes that will be determined by the practitioner according to the circumstances.
- Simultaneous administration:
   ○ component (i) is the U1 snRNA as such and component (ii) is the RNA-based silencing agent.
   ○ component (i) is the U1 snRNA as such as and component (ii) is DNA-based, i.e. it comprises a polynucleotide encoding the silencing RNA molecule.
   ○ component (i) is a polynucleotide encoding the U1 snRNA as such and component (ii) is a RNA-based silencing agent.
   ○ component (i) is a polynucleotide encoding the U1 snRNA as such as and component (ii) is DNA-based silencing agent, i.e. it comprises a polynucleotide encoding the silencing RNA molecule. In this case, both polynucleotides can form part of the same polynucleotide or be provided as separate polynucleotides
- Separate or sequential administration
   ○ component (i) is the U1 snRNA as such and component (ii) is the RNA-based silencing agent.
   ○ component (i) is the U1 snRNA as such as and component (ii) is DNA-based, i.e. it comprises a polynucleotide encoding the silencing agent.
   ○ component (i) is a polynucleotide encoding the U1 snRNA as such and component (ii) is the RNA-based silencing agent.
   ○ component (i) is a polynucleotide encoding the U1 snRNA as such as and component (ii) is DNA-based, i.e. it comprises a polynucleotide encoding the silencing agent. In this case, both polynucleotides must be provided as separate polynucleotides.

The skilled person will appreciate that, wherein both the first and second components are to be used in DNA form, i.e. as polynucleotide encoding the active RNA molecules, the corresponding polynucleotides may be provided in a vector. In the case of separate or sequential delivery, each polynucleotide will form part of a different vector. In the case of simultaneous administration, it is possible to provide both polynucleotides in different vectors or in a single vector comprising both polynucleotides.

Examples of genes that can be used as a target for designing the compositions of the invention include, but are not limited to, oncogenes, genes encoding transcription factors, receptors, enzymes, structural proteins, cytokines, cytokine receptors, lectins, selectins, immunoglobulins, kinases, phosphatases, prions, proangiogenic polypeptides, proteases and proteins involved in the apoptosis process, genes encoding adhesion molecules, genes encoding surface receptors, genes encoding proteins involved in metastasis or in invasive processes of tumor cells, genes encoding growth factors, the multiple drug resistance gene (MDR1), genes encoding lymphokines, cytokines, immunoglobulins, T-cell receptors, MHC antigens, DNA and RNA polymerases, genes involved in metabolic processes such as the synthesis of amino acids, nucleic acids, tumor suppressing genes, 5-lipooxygenase, phospholipase A2, protein kinase C, p53, p16, p21, MMAC1, p73, zac1, C-CAM, BRCA1, Rb, Harakiri, Ad E1B, protease ICE-CED3, IL-2, IL-3, IL-4, IL-5, IL-6, IL-7, IL-8, IL-9, IL-10, IL-11, IL-12, IL-13, IL-14, IL-15, TNF, GMCSF, interferons, CFTR, EGFR, VEGFR, IL-2 receptor, estrogen receptor, members of the Bcl-2 family (Bcl-2 or Bcl-xL), ras, myc, neu, raf erb, src, fins, jun, trk, ret, gsp, hst and abl, amyloid protein precursor, angiostatin, endostatin, METH-1, METH-2, Factor IX, Factor VIII, collagen, cyclin dependent kinase, cyclin D1, cyclin E, WAF 1, cdk4 inhibitor, MTS1, IL-1, IL-2, IL-3, IL-4, IL-5, IL-6, IL-7, IL-8, IL-9, IL-10, IL-11, IL-12, IL-13, IL-14, IL-15, IL-16, IL-17, erythropoietin, G-CSF, GM-CSF, M-CSF, SCF, thrombopoietin, BNDF, BMP, GGRP, EGF, FGF, GDNF, GGF, HGF, IGF-1, IGF-2, KGF, myotrophin, NGF, OSM, PDGF, somatrophin, TGF-beta, TGF-alpha, VEGF, TNF-alpha, TNF-beta, cathepsin K, cytochrome P-450, farnesyltransferase, glutathione-S transferase, heparanase, HMG CoA synthetase, n-acetyltransferase, phenylalanine hydroxylase, phosphodiesterase, carboxy-terminal protease of ras, Notch, telomerase, TNF-converting enzyme, cadherin E, cadherin N, selectin, CD40, ANF, calcitonin, the corticotrophin-releasing factor, glucagon, gonadotropin, the gonadotropin-releasing hormone, growth hormone, the growth hormone-releasing factor, somatotropin, insulin, leptin, luteinising hormone, luteinizing hormone-releasing hormone, PTH, thyroid hormones, thyroid-stimulating hormone, immunoglobulin CTLA4, hemagglutinin, MHC, VLA-4, the kallikrein-kininogen- kinin CD4 system, sis, hst, ras, abl, mos, myc, fos, jun, H-ras, kiras, c-fins, bcl-2, L-myc, c-myc, gip, gsp, HER-2, bombesin receptor, GABA receptor, EGFR, PDGFR, FGFR, NGFR, interleukin receptors, ion channel receptors, leukotriene receptor antagonists, lipoprotein receptors, opioid receptors, substance P receptors, retinoic acid and retinoid receptors, steroid receptors, T-cell receptors, thyroid hormone receptors, TNF receptors, tPA receptors, calcium pump, proton pump, Na/Ca exchanger, MRP 1, MRP2, P170, LRP, cMOAT, transferrin, APC, brca1, brca2, DCC, MCC, MTS1, NF1, NF2, nm23, p53 and Rb.

The compositions of the present invention can be targeted against genes involved in infectious, tumor, neoplastic or neurodegenerative diseases. Accordingly, in another aspect, the invention provides a composition or kit of the invention, a bifunctional polynucleotide of the invention or a vector or a cell comprising the bifunctional polynucleotide for the treatment or the prevention of infectious, tumor, neoplastic or neurodegenerative diseases.

In yet another aspect, the invention provides the use of a composition or kit of the invention, a bifunctional polynucleotide of the invention or a vector or a cell comprising the bifunctional polynucleotide of the invention for the preparation of a medicament for the treatment or the prevention of infectious, tumor, neoplastic or neurodegenerative diseases.

In yet another aspect, the invention provides a method for the treatment of a infectious, tumor, neoplastic or neurodegenerative diseases comprising administering to a patient in need thereof a composition or kit of the invention, a bifunctional polynucleotide of the invention or a vector or a cell comprising the bifunctional polynucleotide of the invention.

In a preferred embodiment, the compositions of the invention can be targeted against genes of pathogenic organisms necessary for the pathogenicity of said organisms as well as against host genes necessary for the pathogen to interact with said host. In this case, the compositions are suitable for the treatment of infectious diseases, including viral and bacterial infections. Thus, in another aspect, the invention relates to a composition or kit of the invention with a bifunctional polynucleotide of the invention, with a vector or a cell of the invention for the treatment or the prevention of infectious diseases. In another embodiment, the invention relates to the use of a composition or kit of the invention with a bifunctional polynucleotide of the invention, with a vector or a cell of the invention for the preparation of a medicament for the treatment or the prevention of infectious diseases.

Viral diseases that can be treated with the compositions of the invention include, without necessarily being limited to, disorders or diseases associated with the corona virus responsible for the Severe Acute Respiratory Syndrome (SARS), herpes simplex virus (HSV), hepatitis B virus (HBV), hepatitis C virus (HCV), human T-cell lymphotropic virus (HTLV) types 1 and 2, human immune deficiency virus (HIV) types 1 and 2, cytomegalovirus, papillomavirus, polyomavirus, adenovirus, Epstein-Barr virus, poxvirus, influenza virus, measles virus, rabies virus, tSendai virus, poliomyelitis virus, coxsackie virus, rhinovirus, reovirus and rubeola virus.

Bacterial diseases (for the sake of simplicity, yeast and fungi disorders and diseases are also included) that can be treated with the compositions of the invention include, but are not necessarily limited to, disorders and diseases associated with *Acinetobacter sp., Aeromonas hydrophila, Alcaligenes faecalis, Bacillus cereus, Bacteroides fragilis, Bacteroides ovatus, Bacteroides ureolyticus, Bacteroides vulgatus, Borrelia burgdorferi, Borrelia vincentii, Brucella abortus, Brucella melitensis, Brucella suis, Campylobacter (Vibrio) fetus, Campylobacter jejuni, Chlamydia spp.,Citrobacter diversus, Citrobacter freundii, Corynebacterium jeikeium, Clostridium botulinum, Clostridium difficile, Clostridium perfringens, Clostridium ramosum, Clostridium sporogenes, Clostridium sp., Clostridium tetani, Corynebacterium diphtheriae, Edwardsiella tarda, Enterobacter aerogenes, Enterobacter cloacae, Enterococcus faecalis, Escherichia coli, Francisella tularensis, Haemophilus influenzae, Helicobacter pylori, Klebsiella oxytoca, Klebsiella ozaenae, Klebsiella pneumoniae, Klebsiella rhinoscleromotis, Leptospira icterohemorrhagiae, Mycobacterium tuberculosis, Mycoplasma spp., Neisseria gonorrhoea, Neisseria meningitidis, Peptostreptococcus anaerobius, Peptostreptococcus asaccharolyticus, Peptostreptococcus magnus, Pneumocystis carinii, Prevotella bivia and Prevotella melaninogenica, Pseudomonas aeruginosa, Pseudomonas fluorescens, Pseudomonas stutzeri, Rickettsia prowazeki, Rickettsia tsutsugumushi, Salmonella typhimurium, Shigella dysenteriae, Shigella flexneri, Shigella sonnei, Staphylococcus aureus, Staphylococcus epidermidis, Streptococcus group C, Streptococcus group G, Staphylococcus haemolyticus, Staphylococcus hominis, Staphylococcus simulans, Staphylococcus warneri, Staphylococcus xylosus, Stenotrophomonas maltophilia, Streptococcus agalactiae, Streptococcus bovis, Streptococcus equinus, Streptococcus pneumoniae, Streptococcus pyogenes, Streptococcus pyogenes, Toxoplasma gondii, Treponema carateneum, Treponema pallidum, Treponema pertenue, Vibrio cholerae, Yersinia enterocolitica, Yersinia pestis* and *Yersinia pseudotuberculosis.*

In another embodiment, the compositions and kits of the invention and the bifunctional polynucleotides of the invention are targeted against genes involved in tumor and/or neoplastic processes thus resulting in active compositions against neoplastic processes and different types of cancer such as hematological cancers (e.g. leukemias or lymphomas), neurological tumours (e.g. astrocytomas or glioblastomas), melanoma, breast cancer, lung cancer, head and neck cancer, gastrointestinal tumours (e.g. stomach, pancreatic or colon cancer), liver cancer, renal cell cancer, genitourinary tumours (e.g. ovarian cancer, vaginal cancer, uterine neck cancer, bladder cancer, testicular cancer and prostate cancer), bone and vascular tumours. Thus, in another respect, the invention relates to a composition or kit of the invention, with a bifunctional polynucleotide of the invention, with a vector or a cell of the invention for the treatment or the prevention of tumor or neoplastic diseases. In another embodiment, the invention relates to the use of a composition or kit of the invention with a bifunctional polynucleotide of the invention, with a vector or a cell of the invention for the preparation of a medical product for the treatment or prevention of tumor or neoplastic diseases.

In another embodiment, the compositions and bifunctional polynucleotides of the invention are targeted against the genes involved in neurodegenerative processes such as retinitis pigmentosa, Alexander's disease, Alpers' disease, Alzheimer's disease, amyotrophic lateral sclerosis, ataxia telangiectasia, Batten disease, bovine spongiform encephalopathy (BSE), Canavan disease, Cockayne syndrome, corticobasal degeneration, Creutzfeldt-Jakob disease, Huntington's disease, dementia associated to HIV, Kennedy's disease, Krabbe disease, Lewy body dementia, Machado-Joseph disease (type 3 spinocerebellar ataxia), multiple sclerosis, multiple systemic atrophy, neuroborreliosis, Parkinson's disease, Pelizaeus-Merzbacher disease, Pick's disease, primary lateral sclerosis, prion diseases, Refsum's disease, Sandhoff disease, Schilder's disease, schizophrenia, Spielmeyer-Vogt-Sjogren-Batten disease (also known as Batten disease), spinocerebellar ataxia, spinal muscular atrophy, Steele-Richardson-Olszewski disease, Tabes dorsalis and the like. Thus, in another respect, the invention relates to a composition or kit of the invention, with a bifunctional polynucleotide of the invention, with a vector or a cell of the invention for the treatment or the prevention of neurodegenerative diseases. In another embodiment, the invention relates to the use of the composition or kit of the invention, with a bifunctional polynucleotide of the invention, with a vector or a cell of the invention for the preparation of a medical product for the treatment or the prevention of neurodegenerative diseases.

In another respect, the invention relates to pharmaceutical preparations comprising the compositions of the invention or the bifunctional polynucleotides of the invention together with one or more acceptable pharmaceutical carriers. The pharmaceutically effective carrier can be solid or liquid. A solid carier can include one or more substances that can also act as flavoring agents, lubricants, solubilizers, suspension agents, fillers, sliding agents, compression agents, binders or tablet disintegrating agents; they can also be encapsulating material. In powders, the carrier is a finely divided solid which is mixed with the finely divided active ingredient. In tablets, the active ingredient is mixed with a carrier having the necessary compression properties in suitable proportions and is compacted to the desired shape and size. The powders and tablets can contain up to 99% of the active ingredient. The suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidone, low melting point waxes and ion exchange resins.

Liquid carriers are used for preparing solutions, suspensions, emulsions, syrups, elixirs and pressurised compositions. The active ingredient can be dissolved or suspended in a pharmaceutically acceptable liquid carrier such as water, an organic solvent, a mixture of both or pharmaceutically acceptable oils or fats. The liquid carrier can contain other pharmaceutically suitable additives such as solubilisers, emulsifiers, surface-active agents, buffers, preservatives, sweeteners, flavoring agents, suspension agents, thickeners, colorings, viscosity regulators, stabilizers or osmoregulators. Suitable examples of liquid carriers for oral or parenteral administration include water (partially containing additives according to the foregoing, for example, cellulose derivatives, possibly a sodium carboxymethyl cellulose solution), alcohols (including monohydric and polyhydric alcohols, for example, glycols) and the derivatives thereof, and oils (for example, fractionated coconut oil and peanut oil). For parenteral administration, the carrier can also be an ester oil such as ethyl oleate and isopropyl myristate. Sterile liquid carriers are useful in sterile compositions in liquid form for parenteral administration. The liquid carrier for the pressurised compositions can be halogenated hydrocarbon or another pharmaceutically suitable propellant.

The liquid pharmaceutical compositions that are sterile solutions or suspensions are suitable for intramuscular, intraperitoneal and subcutaneous injection. Sterile solutions can also be administered intravenously. The compounds can also be administered orally in the form of a liquid or solid composition. Pulmonary administration is also contemplated.

The pharmaceutical composition can be in the form of dosage units, for example, as tablets or capsules. In said form, the composition is subdivided into dosage units containing suitable amounts of the active ingredient. The forms of the dosage units can be packaged compositions, for example, packaged powders, vials, blisters, pre-filled syringes or sachets containing liquid. The form of the dosage units can be, for example, a capsule or a tablet itself, or it can be a suitable number of any of those compositions in packaged form. The dose which must be used in the treatment must be subjectively determined by the doctor.

Furthermore, the compounds of this invention can be used as a solution, cream or lotion by means of a formulation with pharmaceutically acceptable vehicles and applied in the affected area.

Furthermore, the invention contemplates pharmaceutical compositions especially prepared for the administration of the nucleic acids forming the first and second component of the invention or for the administration of the bifunctional polynucleotides of the invention. The pharmaceutical compositions can comprise both the first component and the second component, both if it is based on DNA as well as RNA in naked form, i.e., in the absence of compounds protect the nucleic acids from degradation by the nucleases of the organism, which entails the advantage of eliminating the toxicity associated with the reagents used for the transfection. Suitable routes of administration for the naked compounds include the intravascular, intratumoral, intracranial, intraperitoneal, intrasplenic, intramuscular, subretinal, subcutaneous, mucous, topic or oral route (Templeton, 2002, DNA Cell Biol., 21:857-867). The initial concerns in regard to the capacity of these compounds to induce an immune response when administered naked have been investigated by Heidel et al. (Nat. Biotechnol., 2004, 22:1579-1582). By means of the determination of the plasma interleukin and interferon levels after the intraperitoneal and intravenous administration of siRNAs, no immune response was observed while, at the same time, it was observed that the systematic administration of the siRNA was well tolerated.

In another embodiment, the compositions and polynucleotides of the invention are administered by means of the so-called "hydrodynamic administration" in which the compositions are introduced intravascularly into the organism at high speed and volume, which results in high transfection levels with a more diffuse distribution. A modified version of this technique has made it possible to obtain positive results for silencing through the naked siRNAs of exogenous genes (Lewis et al., 2002, Nat. Gen., 32:107-108; McCaffrey et al., 2002, Nature, 418:38-39) and endogenous genes (Song et al., 2003, Science, Nat. Med., 9:347-351) in multiple organs. It has been shown that the effectiveness of the intercellular access depends directly on the volume of the fluid administered and the speed of the injection (Liu et al., 1999, Science, 305:1437-1441). In mice, the administration has been optimized at values of 1 ml/10 g of body weight in a period of 3-5 seconds (Hodges, et al., 2003, Exp. Opin. Biol. Ther., 3:91-918). The exact mechanism allowing *in vivo* cell transfection with siRNAs after their hydrodynamic administration is not fully known. In the case of mice, it is thought that administration through the tail vein takes place at a rate that exceeds the heart rate and that the administrated fluid accumulates in the superior vena cava. This fluid subsequently accesses the vessels in the organs, and after that, through fenestrations in said vessels, accesses the extravascular space. In this way, the siRNA comes into contact with the cells of the target organism before it is mixed with the blood, thus reducing the possibilities of degradation through nucleases. In a preferred embodiment, both the U1i forming the first component of the composition of the invention and the components based on RNA forming the second component of the composition of the invention can contain modifications in their structure that contribute to increasing their stability. Suitable modifications to reduce the sensibility to nucleases include 2'-O-methyl (Czauderna et al., 2003, Nucleic Acids Res., 31:2705-2716), 2'-fluoropyrimidines (Layzer et al., 2004, RNA, 10:766-771).

The compositions and polynucleotides of the invention can be administered forming part of liposomes, conjugated to cholesterol or conjugated to compounds capable of causing the translocation through cell membranes such as the TAT peptide, derived from the HIV-1 TAT protein, the third helix of the homeodomain of the *D. melanogaster* Antennapaedia protein, the VP22 protein of the herpes simplex virus, arginine oligomers and peptides such as those described in WO07069090 (Lindgren, A. et al., 2000, Trends Pharmacol. Sci., 21:99-103; Schwarze, S.R. et al., 2000, Trends Pharmacol. Sci., 21:45-48, Lundberg, M. et al., 2003, Mol. Therapy, 8:143-150 and Snyder, E.L. and Dowdy, S.F., 2004, Pharm. Res., 21:389-393). Alternatively, the second component of the invention, when it is based on DNA, can be administered forming part of a plasmid vector or of a viral vector, preferably those based on adenoviruses, on adeno-associated viruses or on retroviruses, particularly viruses based on murine leukaemia virus (MLV) or on lentiviruses (HIV, FIV, EIAV).

In another embodiment, in the event that the pharmaceutical composition is formed by a first component and by a second component, the composition may comprise instructions for the simultaneous, sequential or separate use of the first and second components of the composition of the invention. Thus, in another aspect, the invention relates to a composition or kit of the invention comprising instructions for the simultaneous, sequential or separate use of the first and second components of the composition. In yet another aspect, the invention relates to composition or kit of the invention as a combined preparation for simultaneous, separate or sequential use.

A person skilled in the art will appreciate that the dose regimen and the corresponding patients to be treated can be determined according to the present invention. The recommended doses are indicated on the label of the product, enabling the person who prescribes to anticipate the dose adjustments depending on the group of patients under consideration, with information which prevents prescribing the incorrect drug to incorrect patients at the incorrect dose.

The dose regimen will be determined by the doctor and other clinical factors, preferably according to one of the previously described methods. As is well known in medical science, the doses for any patient depends on many factors, including the size of the patient, the body surface area, age, the particular compound that is going to be administered to him or her, sex, time and route of administration, general health and whether other drugs are being administered at the same time. The progress can be observed through periodic evaluation.

The compositions of the invention can be administered in doses of less than 10 mg per kilogram of body weight, preferably less than 5, 2, 1, 0.5, 0.1, 0.05, 0.01, 0.005, 0.001, 0.0005, 0.0001, 0.00005 or 0.00001 mg per kilogram of body weight and less than 200 nmol of the RNA agent, i.e., approximately 4.4 x 10¹⁶ copies per kilogram of body weight or less than 1500, 750, 300, 150, 75, 15, 7.5, 1.5, 0.75, 0.15 or 0.075 nmol per kilogram of body weight. The unit dose can be administered by injection, inhalation or by topical administration. The compositions and bifunctional nucleotides of the invention can be administered directly to the organ in which the target mRNA is expressed, in which case doses between 0.00001 mg and 3 mg per organ, preferably between 0.0001 and 0.001 mg per organ, approximately 0.03 and 3.0 mg per organ, approximately 0.1 and 3.0 mg per organ or between 0.3 and 3.0 mg per organ, are administered.

The dose depends on the severity and response of the condition to be treated, and can vary between several days and several months or until it is observed that the condition abates. The optimum dosage can be determined by carrying out periodic measurements of the concentrations of the agent in the organism of the patient. The optimum dose can be determined from the EC50 values obtained through prior *in vitro* or *in vivo* assays in animal models. The unit dose can be administered once a day or less than once a day, preferably less than once every 2, 4, 8 or 30 days. Alternatively, it is possible to administer an initial dose followed by one or several maintenance doses, generally of a smaller amount than the initial dose. The maintenance regimen can involve treating the patient with dosages ranging between 0.01 µg and 1.4 mg/kg of body weight per day, for example 10, 1, 0.1, 0.01, 0.001 or 0.00001 mg per kilogram of body weight per day. The maintenance doses are preferably administered at most once every 5, 10 or 30 days. The treatment must be continued for a time period that will vary according to the kind of alteration the patient is suffering from, its severity and the condition of the patient. After the treatment, the evolution of the patient must be monitored to determine whether the dose has to be increased in the event that the disease does not respond to the treatment or the dose is reduced if an improvement is observed in the disease or if undesirable side effects are observed.

The daily dose can be administered in a single dose or in two or more doses according to the particular circumstances. If repeated administration or frequent administrations are desired, it is advisable that an administration device such as a pump, a semi-permanent catheter (intravenous, intraperitoneal, intracisternal or intracapsular) or a reservoir be implanted.

In another aspect, the invention relates to *in vitro* and *in vivo* non-therapeutic methods for the post-transcriptional inhibition of the expression of a target gene which comprises putting the compositions or bifunctional nucleotides of the invention in contact with a biological model. The biological model is preferably a cell culture or an animal.

In the case of compositions and polynucleotides suitable for their application on a cell culture, there are different procedures for achieving that the polynucleotides access the inside of the cell. On the one hand, it is possible to put the cells in contact with the naked polynucleotides. Putting the cells in contact with the polynucleotides, although it allows the access to the endosomal compartment, does not allowing reach the cytoplasm in functional state (Lingor, P. et al., 2004, Biochem. Biophys. Res. Commun., 315:1123-1133). Therefore, the polynucleotides are preferably administered by means of microinjection or electroporation. Alternatively, it is possible to use chemical transfection using suitable reagents for the transfection of DNA and RNA polynucleotides, particularly, cationic liposomes, peptides permeable to membranes, atelocollagen, hybrid organic-inorganic nanoparticles formed by polymers of polyaspartic polyethylene glycol with calcium phosphate (Kakizawa et al., 2004; J. Control Rel., 97:345-356; Minakuchi et al., 2004, Natl. Acids Res., 32:109 and Muratovska and Eccles, 2004, Fed. Euro. Biochem. Soc. Lett., 558:63-68). In another embodiment, the polynucleotides of the invention can be administered using expression vectors. Obviously, this approach is only possible when the second component of the composition of the invention is based on DNA.

Alternatively, it is possible to modify the agents forming the composition of the invention with chemical moieties that allow an increase in their capture by cells. Thus, the agents forming the invention can be conjugated with different types of compounds such as peptides and organic compounds. The conjugation can be carried out by means of methods known to a person skilled in the art, including the methods of Lambert et al, (Drug Deliv., 2001, Rev.: 47:99-112), in which nucleic acids are coupled to nanoparticles of polyalkylcyanoacrylate (PACA); Fattal et al., (J. Control Release 1998, 53:137-143) which describes nucleic acids coupled to nanoparticles; Schwab et al., (Ann. Oncol., 1994, 5 Suppl. 4:55-58) which describes nucleic acids unique to intercalating agents, hydrophobic groups, polycations or PACA nanoparticles and Godard et al., (Eur. J. Biochem., 1995, 232:404-410) which describes nucleic acids coupled to nanoparticles.

In another embodiment, the agents of the invention connect are coupled to lipophilic moieties which can include a cationic group and can be associated with one or the two strands of the expression silencing-agent. Suitable lipophilic agents include cholesterol, vitamin E, vitamin K, vitamin A, folic acid, a cationic coloring (for example Cy3), cholic acid, acetic adamantine, 1-pyrenebutyric acid, dihydrotestosterone, 1,3-bis-O (hexadecyl) glycerol, a geranyloxyhexyl group, hexadecylglycerol, borneol, menthol, 1,3-propanediol, heptadecyl, palmitic acid, myristic acid, O3-(oleoyl) lithocholic acid, 03-(oleoyl) cholenic acid, dimethoxytrityl or phenoxazine.

The effects of the silencing can be observed by means of the detection of phenotype changes or by means of the use of biochemical techniques allowing the detection of changes in the expression levels of a certain mRNA or of the proteins encoded by it, such as RNA hybridization, nuclease protection, Northern hybridization, monitoring of genetic expression by means of DNA microarrays, Western blotting, RIA, ELISA and FACS. In animal or cultured cell models, it is possible to detect the modification of the expression of a reporter gene or a gene of resistance to a toxic agent the products of which are easily detectable. Suitable reporter genes include acetohydroxyacid synthase (AHAS), alkaline phosphatise (AP), beta galactosidase (LacZ), beta glucuronidase (GUS), chloramphenicol acetyltransferase (CAT), fluorescent green protein (GFP), horse-radish peroxidise (HRP), luciferase (Luc), nopaline synthase (NOS), octopine synthase (OCS) and the derivatives thereof. Suitable selection markers include genes encoding resistance to ampicillin, bleomycin, chloramphenicol, gentamycin, hygromycin, kanamycin, lincomycin, methotrexate, phosphinotricin, puromycin and tetracycline.

Alternatively, the non-therapeutic method for silencing post-transcriptional gene expression can be applied to an animal model in which the gene the expression of which is to be silenced is expressed. In this case, it is necessary for the gene expression-silencing agent to access the tissue or organ in the animal in which a post-transcriptional silencing of gene expression is to be caused. To that end, any of the previously described methods for the therapeutic administration of the agents of the invention to a patient can be used. In a preferred embodiment, the invention contemplates the administration of compositions targeted against different target mRNAs encoding proteins involved in the same functional pathway. Functional pathways that can be the object of inhibition by means of the use of combinations of compositions of the invention including, but not necessarily limited to, glucolysis, gluconeogenesis, Krebs cycle, the pentose phosphate pathway, synthesis of glycogen, Calvin's cycle, degradation of triacylglycerides, activation of fatty acids, beta oxidation, de novo synthesis of fatty acids, synthesis of cholesterol, urea cycle, shikimate pathway, synthesis of amino acids, oxidative phosphorylation, photosynthesis, synthesis of purines, synthesis of pyrimidines, histidine metabolism, porphyrin metabolism, inositol metabolism, and the like. A person skilled in the art will appreciate that the different steps of the aforementioned pathways as well as the enzymes responsible for each of said stages are known, therefore the development of U1is and other expression-silencing agents specific for one or several genes involved in the steps of said process can be carried out by means of routine experiments if the aforementioned criteria for choosing the target sequences in the pre-RNAms and the RNAms having a greater possibility of being recognised by U1is or similar silencing agents.

Gene therapy, which is based on inserting therapeutic genes into cells by means of *ex vivo* or *in vivo* techniques, is one of the most important applications of gene transfer. In the literature, suitable vectors and methods have been described for *in vitro* or *in vivo* gene therapy have been described and are known to the person skilled in the art; see, for example, Giordano, Nature Medicine 2 (1996), 534-539; Schaper, Circ. Res 79 (1996), 911-919; Anderson, Science 256 (1992), 808-813; Isner, Lancet 348 (1996), 370-374; Muhlhauser, Circ. Res 77 (1995), 1077-1086; Wang, Nature Medicine 2 (1996), 714-716; WO94/29469; WO97/00957 or Schaper, Current Opinion in Biotechnology 7 (1996), 635-640 and the references mentioned therein. The gene can be designed for its direct insertion or for its insertion by means of liposomes or viral vectors (for example, adenoviral vectors, retroviral vectors) in the cell. Preferably, said cell is a non-human germinal line cell, non-human embryonic cell or a non-human egg cell or a cell derived therefrom. More preferably said cell is an adult stem cell or a non-human embryonic stem cell. As is clear from the foregoing, it is preferred that in the use of the invention, the nucleic acid sequence be operatively bound to the regulatory elements allowing the expression and/or the integration of the polypeptides of the invention in specific cells. Suitable gene distribution systems that can be used according to the invention may include liposomes, receptor-mediated distribution systems, naked DNA and viral vectors such as the herpes virus, the retrovirus, the adenovirus and adeno-associated viruses, among others. The distribution of nucleic acids to a specific site in the body for gene therapy can also be achieved by using a biolistic distribution system, such as that described by Williams (Proc. Natl. Acad. Sci. USA, 88 (1991), 2726-2729). The standard methods for transfecting cells with recombinant DNA are well known to a person skilled in the art of molecular biology, see, for example, WO94/29469; see also *supra.* Gene therapy can be carried out by directly administering the recombinant DNA molecule or the vector of the invention to a patient or by transfecting the cells with the polynucleotide or the vector of the invention *ex vivo* and administering the transfected cells to the patient.

Particularly preferred gene therapy vectors in the context of the present invention are parvoviral vectors. Thus, in this preferred aspect the invention relates the use of animal parvoviruses, in particular dependoviruses such as infectious human or simian AAV, and the components thereof (e.g., an animal parvovirus genome) for use as vectors for introduction and/or expression of a composition of kit of the invention.

Viruses of the Parvoviridae family are small DNA animal viruses. The family Parvoviridae may be divided between two subfamilies: the Parvovirinae, which infect vertebrates, and the Densovirinae, which infect insects. Members of the subfamily Parvovirinae are herein referred to as the parvoviruses and include the genus Dependovirus. As may be deduced from the name of their genus, members of the Dependovirus are unique in that they usually require coinfection with a helper virus such as adenovirus or herpes virus for productive infection in cell culture. The genus Dependovirus includes AAV, which normally infects humans (e.g., serotypes 2, 3A, 3B, 5, and 6) or primates (e.g., serotypes 1 and 4, which are thought to have been originated from monkeys, but also infect humans), and related viruses that infect other warm-blooded animals (e.g., bovine, canine, equine, and ovine adeno-associated viruses). Further information on AAV serotypes and on strategies for engineering hybrid AAV vectors derived from AAV serotypes is described in Wu et al. (2006, Molecular Therapy 14:316-327). It is understood that the invention is not limited to AAV but may equally be applied to hybrid AAV vectors derived from two or more different AAV serotypes and to other parvoviruses and hybrids thereof. Techniques regarding the use of AAV vectors in gene therapy are described in, for example, WO2007/046703 and WO2007/148971.

A composition or polynucleotide of the invention may thus be delivered using a "recombinant parvoviral or AAV vector" ("rAAV vector") which herein may refer to a vector comprising a polynucleotide of the invention sequences of interest that is flanked by at least one parvoviral or AAV inverted terminal repeat sequences (ITRs). A composition of the invention may be delivered using two rAAV vectors each one encoding one of the components of such a composition. rAAV vectors suitable for use in the invention can be replicated and packaged into infectious viral particles when present in an insect host cell that is expressing AAV rep and cap gene products (i.e. AAV Rep and Cap proteins). When an rAAV vector is incorporated into a larger nucleic acid construct (e.g. in a chromosome or in another vector such as a plasmid or baculovirus used for cloning or transfection), then the rAAV vector is typically referred to as a "pro-vector" which can be "rescued" by replication and encapsidation in the presence of AAV packaging functions and necessary helper functions.

The invention is described below by means of the following examples which are merely illustrative and by no means limiting on the scope for the invention.

### EXAMPLES

### Example 1.

### 1.1 Reagents

### Listing of shRNAs/miRNAs used in the experiments:

| **Identification key** | **Present in SEQ ID NO** | **Target sequence in mRNA** | **Target gene** |
|---|---|---|---|
| shRNAαLuc154 | 1 | ACTTACGCTGAGTACTTCGAA | Luciferase |
| shRNAαLuc158 | 2 | ACGCTGAGTACTTCGAAATGT | Luciferase |
| shRNAαLuc163 | 3 | GAGTACTTCGAAATGTCCGTT | Luciferase |
| shRNAαLuc1546 | 4 | GTTGTGTTTGTGGACGAAGTA | Luciferase |
| shRNA1αNotch1 | 5 | TGGCGGGAAGTGTGAAGCG | Notch1 |
| shRNA2αNotch1 | 6 | ACACCAACGTGGTCTTCAA | Notch1 |

### Sequences of the shRNAs/miRNAs

A: 5' Sequence; B: Sense (transient strand); C: Loop; D: Antisense (guide strand); E: Termination signal.
SEQ ID NO 1-shRNAαLuc154 
CCGGACTTACGCTGAGTACTTCGAACTCGAGTTCGAAGTACTCAGCGTAAGTTTTTTG
A B C D E
SEQ ID NO 2-shRNAαLuc158 
CGGACGCTGAGTACTTCGAAATGTCTCGAGACATTTCGAAGTACTCAGCGTTTTTTG
A B C D E
SEQ ID NO 3-shRNAαLuc163 
CGGGAGTACTTCGAAATGTCCGTTCTCGAGAACGGACATTTCGAAGTACTCTTTTTG
A B C D E
SEQ ID NO 4-shRNAαLuc1546 
CCGGGTTGTGTTTGTGGACGAAGTACTCGAGTACTTCGTCCACAAACACAACTTTTTG
A B C D E
SEQ ID NO 5- shRNA1αNotch1 
GATCCCCTGGCGGGAAGTGTGAAGCGTTCAAGAGACGCTTCACACTTCCCGCCATTTTTGGAAA
A B C D E
SEQ ID NO 6- shRNA1αNotch1

GATCCCCACACCAACGTGGTCTTCAATTCAAGAGATTGAAGACCACGTTGGTGTTTTTTGGAAA
A B C D E

Sequences 1 to 4 were obtained from Sigma. Sequence 5 (described by Purow et al. Cancer Res. (2005) 65:2353-63) and 6 were obtained in the laboratory by means of the cloning in plasmid pSuper (Brummelkamp et al. Science (2002) 296: 550-3) of an oligonucleotide containing the sequences:
SEQ ID NO: 5
   GATCCCCTGGCCGGGAAGTGTGAAGCGTTCAAGAGACGCTTCACACTTCCCGCCATTTTTGGAAA
SEQ ID NO: 6
   GATCCCCACACCAACGTGGTCTTCAATTCAAGAGATTGAAGACCACGTTGGTGTTTTTTGGAAA

In all of the cases, the sequences are cloned in the *BglII-HindII* site of plasmid pSuper designed to express them when they are transfected inside the cell. The expression of the shRNA is produced in the nucleus of the cell, from where it is transported to the cytoplasm, where it is processed to give rise to a siRNA. This interacts with the silencing machinery that activates it to recognise the target mRNA by base pairing and to activate its degradation. In sequences 1-5 the target RNA is firefly luciferase [*Photinus pyralis* (GenBank Accession No. M15077)]. Sequences 6 and 7 are aimed against the Notch1 gene (NP_060087.3). The sequence of all the clones was verified by an ABI Prism 310 sequencer (Applied Biosystems). The plasmid DNA was purified with a Maxiprep kit (Marlingen) before the transfection.

### List of U1/U1i snRNAs used in the experiments:

| **Target Gene** | **Identification Key** | **SEQ ID NO:** | **U1i sequence matching the target mRNA** |
|---|---|---|---|
| - | U1αMock | 7 | CCUGCCAGGUAAGUA ⁽¹⁾ |
| Luciferase | U1iαLuc | 8 | CCUGCCA**U**G**G**AA**C**UA ⁽²⁾ |
| Notch1 | U1iαNotch1 | 9 | CCU**UUUGCUCUGCCU** |

### LITERATURE REFERENCES:

(1) Fortes P. et al., (Proc. Natl. Acad. Sci. USA, 2003 100:8264-8269)
(2) Fortes P. *et al*., (Proc. Natl. Acad. Sci. USA, 2003 100:8264-8269 and WO02083908)

A U1 snRNA (U1αMock) containing a sequence identical to that of the endogenous U1 snRNA at its 5' end with 4 point mutations in loop3 was used as control. Two cytosines hybridizing with two guanosines in this loop were interchanged with one another. The resulting molecule does not have any functional difference with the endogenous U1 snRNP. Previously, it had been proved that upon adding a sequence hybridizing with the endogenous U1 snRNA in the 3'UTR part of a reporter gene, an inhibition in the expression of this reporter gene occurred. This is the proof of concept that the system works.

The UliaLuc was constructed from the U1WT sequence. In this sequence, three positions (in bold and underlined) were mutated. It was observed that upon co-transfecting a modified U1 snRNA with a reporter gene containing the target for said U1 snRNP, the expression of the reporter gene was inhibited. Numerous constructs were tested in different positions of the 3'UTR and by means of numerous *in vitro* studies a series of conditions for designing different modified U1 snRNAs was established. These rules are:
- U1 snRNA inhibits only when it is binds to 3' terminal exon of the target mRNA
- U1 snRNA specifically inhibits those mRNAs that are polyadenylated by the classic polyadenylation machinery
- several U1 snRNAs act in a synergic manner
- the binding between the U1 snRNA and its target has to be at least 7nt to inhibit its expression.
- if the 5' area of U1 snRNAs is extended, this U1 snRNA inhibits worse.
- the accessibility of the target sequence affects the inhibition mediated by U1 snRNA

The U1αNotch1 sequence is completely different from mock or luciferase sequences and originated by mutagenizing the first 12 nucleotides of U1.

### 1.2 Experimental model of the inhibition of the expression of the luciferase gene.

The HeLa cells (obtained from ATCC) were grown in the DMEM medium, supplemented with 10% BFS (bovine fetal serum) and 1% streptomycin, in an environment with 5% CO2. All the culture reagents came from Invitrogen. All the plasmids employed were transfected in the cell using calcium phosphate according to Aparicio et al. (J. Virol., 1006, 80:12236-47). Furthermore, a control plasmid expressing renilla luciferase (pSV-RL, Promega) and another expressing firefly luciferase were transfected with the target sequences of siRNAs and U1s (pGL-3, Promoter, Promega). The cell extracts were collected 48 hours after transfection and the expression of the luciferase was quantified by means of measuring their activity (Dual Luciferase System, Promega) according to Aparicio et al. 2006 J. Virol. 80:12236-47., in an Orion II plate luminometer (Berthold).

### 1.3 Experimental model of the inhibition of the expression of the endogenous Notch gene.

HeLa cells were transfected using Lipofectamine 2000 (Invitrogen) with pGEM plasmids (Promega) expressing UliaNotch1 or U1αMock as a control or shRNA1αNotch1 or shRNA2aNotch1. The cell extracts were collected 48 and 72 hours after transfection and the accumulation of Notch1 with specific antibodies (C-20 sc-6014-R, Santa Cruz Biotechnology) was quantified by means of Western blot developed by chemiluminescence (Perkin Elmer) and quantified (Image Quant ECL, Amersham). The actin levels were also quantified as a load control in each case. Notch1 activity was also measured upon measuring its capacity to translocate the NFκB nucleus. The NFκB activity was quantified by measuring its capacity to activate the expression of luciferase starting from a plasmid in which the luciferase gene is under an promoter inducible by NFκB (Stratagene). When this system was used, the luciferase was measured as has been previously described.

### Example 2.

### The effect of the different snRNA U1s and interference RNAs on the expression of the luciferase gene.

The inhibition was tested in the presence of a dose or half a dose of shRNA against luciferase, a dose or half a dose of U1iαLuc or a mixture of half a dose of shRNA and half a dose of U1iαLuc. The results (Figures 2-5) showed that there was synergy, given that the inhibition obtained using both techniques is greater than the inhibition resulting from using each system separately. Furthermore, the synergy is robust, given that the inhibition obtained with half a dose of U1i + half a dose of shRNA is greater than the inhibition obtained with a dose of the same U1i or a dose of the same shRNA. The synergy exists independently of the inhibitory capacity of the shRNA used, given that it is detected with strong shRNAs, i.e., which induce inhibitions of between 10 and 20 times (Figures 2-3), medium snRNAs, which induce inhibitions 4 times (Fig. 4) and weak shRNAs, which induce inhibitions of 2 times (Figure 5). Furthermore, the existence of synergy permits allows reducing the dose of shRNA or the dose of the U1i between 8 and 20 times, as much as if a strong shRNA (Figure 6) or a medium shRNA (Figure 7) is used, maintaining a greater inhibition than that obtained with the maximum dose of shRNA or U1i.

### Example 3.

### The effect of the different shRNA U1s and interference RNAs on the expression of the Notch1 gene.

### 3.1 U1 snRNA in combination with mature interference RNAs (shRNAs)

It would be possible that this synergy in the inhibition would work only with exogenous genes, co-transfected with the inhibitors. To find out if there was inhibitory synergy against endogenous genes, the inhibition of Notch1 has been analyzed. Notch1 has been determined by Western blot (Figure 8A) and by its capacity to reduce the amount of nuclear NFκB. In this latter case, a plasmid has been used in which the luciferase gene is under a promoter inducible by NFκB. (Figure 8B). The inhibition has been tested in the presence of a dose or half a dose of shRNAs against Notch1, a dose (8 micrograms) or half a dose (4 micrograms) of U1iαNotch1 or a mixture of half a dose of shRNA and half a dose of U1iαNotch1. The result shows that there is synergy, since the inhibition obtained using both techniques is greater than the inhibition resulting from the use of each system separately (Figure 8). Furthermore, the synergy is robust, given that the inhibition obtained with half a dose of U1i + half a dose of shRNA is greater than the inhibition obtained with a dose of the same U1i or a dose of the same shRNA.

## Claims

1. A composition comprising one or several containers or a kit-of-parts comprising:
(i) a first component comprising at least an U1 snRNA or a polynucleotide encoding an U1 snRNA, wherein said U1 snRNA is modified in its binding sequence to the GU consensus sequence of the 5' end of the intron such that it binds specifically to a pre-selected region of the 3' terminal exon of a target pre-mRNA and which is capable of inhibiting the processing of said target pre-mRNA; and
(ii) a second component comprising at least one gene expression-silencing agent targeted specifically at a pre-selected region of the mRNA resulting from the processing of the pre-mRNA which is the target of the U1 snRNA as defined in (i) and which is capable of causing the *in vivo* silencing of said target mRNA.

2. A composition or a kit according to claim 1, wherein the silencing agent comprised within the second component is selected from the group consisting of:
(i) siRNA
(ii) miRNA
(iii) shRNA
(iv) a polynucleotide encoding an agent according to (i), (ii) or (iii), and
(v) a combination of one or several agents according to (i) to (iv).

3. A composition or a kit according to claim 2, wherein component (ii) is a polynucleotide comprising a sequence encoding at least one shRNA.

4. A composition or a kit according to any of claims 1 to 3 wherein component (i) comprises several polynucleotides encoding U1 snRNAs modified in their binding sequences to the GU consensus sequence of the 5' end of the intron so that they bind specifically to different pre-selected regions of the same target pre-mRNA and/or component (ii) comprises several gene expression-silencing agent targeted specifically at different pre-selected region of the mRNA resulting from the processing of the pre-mRNA which is the target of the U1 snRNA as defined in (i) and which are capable of causing the *in vivo* silencing of said target mRNA.

5. A composition or a kit according to any of claims 1 to 4, wherein the second component is a polynucleotide encoding a silencing agent and wherein said polynucleotide and the polynucleotide forming the first component form part of a single vector.

6. A composition or a kit according to claim 5, wherein said single vector is a viral vector.

7. A composition or a kit according to claim 6, wherein the viral vector is selected from the group consisting of adenoviruses, adeno-associated viruses, retroviruses, lentiviruses, herpes viruses, SV40 and alphaviruses.

8. A polynucleotide comprising:
(i) a sequence encoding at least one U1 snRNA modified in its binding sequence to the GU consensus sequence of the 5' end of the intron such that it binds specifically to a pre-selected region of the 3' terminal exon of a target pre-mRNA and which is capable of inhibiting the processing of the target pre-mRNA, and
(ii) a sequence encoding a silencing agent targeted specifically at a pre-selected region of the mRNA resulting from the processing of the pre-mRNA which is the target of the U1 snRNA encoded by the polynucleotide defined in (i) and which is capable of causing the *in vivo* silencing of said target mRNA.

9. A polynucleotide as defined in claim 8 wherein the sequence encoding a silencing agent comprises the sense and antisense strands of at least one siRNA, encodes at least one pre-miRNA or encodes at least one shRNA.

10. A polynucleotide according to claims 8 or 9, wherein the sequences (i) and (ii) are operatively coupled to expression-regulating regions.

11. A polynucleotide according to claim 10, wherein the expression regulating-sequences of the sequences (i) and (ii) are different.

12. An expression vector comprising a polynucleotide according to any of claims 8 to 11.

13. A vector according to claim 12, wherein the expression vector is a viral vector.

14. A vector according to claim 13, wherein the viral vector is selected from the group consisting of adenoviruses, adeno-associated viruses, retroviruses, lentiviruses, herpes viruses, SV40 and alphaviruses.

15. A cell comprising a vector according to claims 12 to 14.

16. A non-human transgenic animal comprising a polynucleotide according to claims 8 to 11 integrated in its genome , a vector according to any of claims 12 to 14 or a cell according to claim 15.

17. A composition or kit according to any of claims 1 to 7, a polynucleotide according to any of claims 8 to 11, a vector according to any of claims 12 to 14 or a cell according to claim 15 for use in medicine or for use as a medicament.

18. A composition or kit according to any of claims 1 to 7, a polynucleotide according to any of claims 8 to 11, a vector according to any of claims 12 to 14 or a cell according to claim 15 for the treatment or the prevention of infectious, tumor, neoplastic or neurodegenerative diseases.

19. Use of a composition or kit according to any of claims 1 to 7, a polynucleotide according to any of claims 8 to 11, a vector according to any of claims 12 to 14 or a cell according to claim 15 for the preparation of a medicament for the treatment or the prevention of infectious, tumor, neoplastic or neurodegenerative diseases.

20. A method for the treatment of a infectious, tumor, neoplastic or neurodegenerative diseases comprising administering to a patient in need thereof a composition or kit according to any of claims 1 to 7, a polynucleotide according to any of claims 8 to 11, a vector according to any of claims 12 to 14 or a cell according to claim 15.

21. A composition or kit according to claim 18, a use according to claim 19 or a method as defined in claim 20 wherein the infectious disease is a infectious disease caused by a virus.

22. A composition, kit or method according to claim 21 wherein the infectious disease caused by a virus is an infection by HBV, HCV or HIV.

23. A pharmaceutical composition comprising a composition or kit according to any of claims 1 to 7, a polynucleotide according to any of claims 8 to 11, a vector according to any of claims 12 to 14 or a cell according to claim 15 and a pharmaceutically acceptable carrier.

24. A composition or kit according to any of claims 1 to 7 comprising instructions for the simultaneous, sequential or separate use of the first and second components.

25. A composition or kit according to any of claims 1 to 7 as a combined preparation for simultaneous, separate or sequential use.

26. A non-therapeutic method for the post-transcriptional inhibition *in vitro* or *in vivo* of the expression of a target gene in a biological system comprising putting said system in contact with a composition according to any of claims 1 to 7, with a polynucleotide according to any of claims 8 to 11, with a vector according to any of claims 12 to 14 or with a cell according to claim 15.

27. A method according to claim 26, wherein said biological model is an animal model.

28. A method according to claims 26 or 27, wherein the post-transcriptional inhibition includes the inhibition of two or more target mRNAs involved in the same functional pathway.


SEQUENCE LISTING

<110> PROYECTO DE BIOMEDICINA CIMA, S.L.

<120> METHODS AND COMPOSITIONS CAPABLE OF CAUSING A POST-TRANSCRIPTIONAL
      SILENCING OF GENE EXPRESSION IN A SYNERGIC MANNER

<130> P3574EPPC

<140> EP 09705069.4
<141> 2009-01-29

<150> P200800222.5
<151> 2008-01-29

<160> 9

<170> Patent In version 3.4

<210> 1
<211> 58
<212> DNA
<213> Artificial sequence

<220>
<223> shRNAalphaLuc154
<400> 1
ccggacttac gctgagtact tcgaactcga gttcgaagta ctcagcgtaa gtttttg        58

<210> 2
<211> 57
<212> DNA
<213> Artificial sequence

<220>
<223> shRNAalphaLuc158

<400> 2
cggacgctga gtacttcgaa atgtctcgag acatttcgaa gtactcagcg ttttttg        57

<210> 3
<211> 57

<212> DNA
<213> Artificial sequence

<220>
<223> shRNAalphaLuc163

<400> 3
cgggagtact tcgaaatgtc cgttctcgag aacggacatt tcgaagtact ctttttg          57

<210> 4
<211> 58
<212> DNA
<213> Artificial sequence

<220>
<223> shRNAalphaLuc1546

<400> 4
ccgggttgtg tttgtggacg aagtactcga gtacttcgtc cacaaacaca actttttg         58

<210> 5
<211> 64
<212> DNA
<213> Artificial sequence

<220>
<223> shRNA1alphaNotch1

<400> 5
gatcccctgg cgggaagtgt gaagcgttca agagacgctt cacacttccc gccatttttg       60

gaaa                                                                    64
<210> 6
<211> 64
<212> DNA
<213> Artificial sequence

<220>
<223> shRNA1alphaNotch1

<400> 6
   gatccccaca ccaacgtggt cttcaattca agagattgaa gaccacgttg gtgttttttg      60
   gaaa                                                                  64

<210> 7
<211> 607
<212> DNA
<213> Artificial sequence

<220>
<223> Plasmid control pGem3z+:U1-Mscl (wild, not-endogenous snRNA U1).
      Incorporates the sequence for the transcription of an endogenous
      snRNA U1 modified in 4 positions of its 3' end.
      Codifies U1aMock

<220>
<221> Promoter
<222> (9)..(400)

<220>
<221> mic_feature
<222> (401)..(469)
<223> Transcription sequence of snRNA U1-Mscl

<220>
<221> modified_base
<222> (489)..(499)
<223> Modified bases with regard to an endogenous snRNA U1

<220>
<221> modified_base
<222> (509)..(510)
<223> Modified bases with regard to an endogenous snRNA U1

<220>
<221> terminator
<222> (570)..(602)
<223> Termination sequence of the transcription

<400> 7
ggatccggta aggaccagct tctttgggag agaacagacg caggggcggg agggaaaaag      60
ggagaggcag acgtcacttc cccttggcgg ctctggcagc agattggtcg gttgagtggc      120
agaaaggcag acggggactg ggcaaggcac tgtcggtgac atcacggaca gggcgacttc      180
tatgtagatg aggcagcgca gaggctgctg cttcgccact tgctgcttca ccacgaagga      240
gttcccgtgc cctgggagcg ggttcaggac cgctgatcgg aagtgagaat cccagctgtg      300
tgtcagggct ggaaagggct cgggagtgcg cggggcaagt gaccgtgtgt gtaaagagtg      360
aggcgtatga ggctgtgtcg gggcagaggc ccaagatctc atacttacct ggcaggggag      420
ataccatgat cacgaaggtg gttttcccag ggcgaggctt atccattgca ctccggatgt      480
gctgacccct gcgatttggc caaatgtgcc aaactcgact gcataatttg tggtagtggg      540
ggactgcgtt cgcgctttcc cctgactttc tggagtttca aaagtagact gtacgctaac      600
cggatcc                                                                607

<210> 8
<211> 607
<212> DNA
<213> Artificial sequence

<220>
<223> Inhibition plasmid of reporter genes pGem3z+:U1-Mut. Derived from the
      plasmid pGem3z+:U1-Mscl by modification of 3 bases in the 5′ end of
      snRNA U1. Codifies and transcribes U1iaLuc.

<220>
<221> modified_base
<222> (404)..(404)

<220>
<221> modified_base
<222> (407)..(407)

<220>
<221> modified_base
<222> (409)..(409)

<400> 8
ggatccggta aggaccagct tctttgggag agaacagacg caggggcggg agggaaaaag      60
ggagaggcag acgtcacttc cccttggcgg ctctggcagc agattggtcg gttgagtggc      120
agaaaggcag acggggactg ggcaaggcac tgtcggtgac atcacggaca gggcgacttc      180
tatgtagatg aggcagcgca gaggctgctg cttcgccact tgctgcttca ccacgaagga      240

gttcccgtgc cctgggagcg ggttcaggac cgctgatcgg aagtgagaat cccagctgtg 300
tgtcagggct ggaaagggct cgggagtgcg cggggcaagt gaccgtgtgt gtaaagagtg 360
aggcgtatga ggctgtgtcg gggcagaggc ccaagatctc atagttccat ggcaggggag 420
ataccatgat cacgaaggtg gttttcccag ggcgaggctt atccattgca ctccggatgt 480
gctgacccct gcgatttggc caaatgtgcc aaactcgact gcataatttg tggtagtggg 540
ggactgcgtt cgcgctttcc cctgactttc tggagtttca aaagtagact gtacgctaac 600
cggatcc 607

<210> 9
<211> 607
<212> DNA
<213> Artificial sequence

<220>
<223> Plasmid for the inhibition of Notch pGem3z+:U1-Notch. Derived from
      plasmid pGem3z+:U1-Mscl by modification of the bases at the 5' end of
      the snRNA U1. Codifies U1iaNotch1.

<400> 9
ggatccggta aggaccagct tctttgggag agaacagacg caggggcggg agggaaaaag 60
ggagaggcag acgtcacttc cccttggcgg ctctggcagc agattggtcg gttgagtggc 120
agaaaggcag acggggactg ggcaaggcac tgtcggtgac atcacggaca gggcgacttc 180
tatgtagatg aggcagcgca gaggctgctg cttcgccact tgctgcttca ccacgaagga 240
gttcccgtgc cctgggagcg ggttcaggac cgctgatcgg aagtgagaat cccagctgtg 300
tgtcagggct ggaaagggct cgggagtgcg cggggcaagt gaccgtgtgt gtaaagagtg 360
aggcgtatga ggctgtgtcg gggcagaggc ccaagatctc aaggcagagc aaaaggggag 420
ataccatgat cacgaaggtg gttttcccag ggcgaggctt atccattgca ctccggatgt 480
gctgacccct gcgatttggc caaatgtgcc aaactcgact gcataatttg tggtagtggg 540
ggactgcgtt cgcgctttcc cctgactttc tggagtttca aaagtagact gtacgctaac 600
cggatcc 607